(19) Europäisches Patentamt
European Patent Office
Office européen des brevets

(11) **EP 3 985 105 A1**

(12) **EUROPEAN PATENT APPLICATION**

(43) Date of publication:
**20.04.2022 Bulletin 2022/16**

(21) Application number: **20202376.8**

(22) Date of filing: **16.10.2020**

(51) International Patent Classification (IPC):
***C12N 5/0783*** (2010.01)

(52) Cooperative Patent Classification (CPC):
**C12N 5/0637;** C12N 2501/2302; C12N 2502/1382

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Uniwersytet Gdanski**
**80-309 Gdansk (PL)**
• **Gdanski Uniwersytet Medyczny**
**80-210 Gdansk (PL)**

• **PolTREG S.A**
**80-855 Gdansk (PL)**

(72) Inventors:
• **Marek-Trzonkowska, Natalia**
**80-752 Gdansk (PL)**
• **Piekarska, Karolina**
**80-282 Gdansk (PL)**

(74) Representative: **Pawlowska, Justyna**
**Kancelaria Patentowa Justyna Pawlowska
Wzgorze Bernardowo 263B/6
81-583 Gdynia (PL)**

(54) **CONDITIONED REGULATORY T CELL POPULATION TREGS WITH ENHANCED THERAPEUTIC POTENTIAL, METHOD FOR OBTAINING OF CONDITIONED TREGS AND THE MEDICAL USE OF THE TREGS POPULATION**

(57)      Invention relates to population of CD4$^+$FoxP3$^+$ regulatory T cells (Tregs) characterized by high expression of CD25 and enhanced immunosuppressive function while the population is obtained as a result of coculture in indirect contact with adipose tissue derived mesenchymal stem cells (ASCs) from allogeneic donors wherein Tregs. ASCs are cocultured in indirect contact in two compartments separated from each other with a membrane with pore size ≤0.4μm impermeable for cells and extracellular vesicles with the diameter >0.4 μm but permeable for secreted soluble molecules that pass freely through the membrane between the two culture compartments.

**EP 3 985 105 A1**

**Description**

The invention relates to a conditioned regulatory T cell population (Tregs) characterized by enhanced immunosuppressive potential (functionality) and a method of the potentiated Treg population production in vitro and their medical use in therapy of adverse immune responses, especially such as graft rejection, allergic reactions, graft versus host disease (GVHD), exaggerated inflammatory responses and autoimmune diseases, including e.g. multiple sclerosis (MS) or rheumatoid arthritis (RA).

Tregs are a unique leukocyte subset that does not protect from infectious agents but controls activation of other immune cells. Despite Tregs account for ≈1% of peripheral blood lymphocytes they maintain self-tolerance (1). Lack of Tregs that is characteristic for immune dysregulation, polyendocrinopathy, enteropathy, X-linked syndrome (IPEX) leads to development of multiple autoimmune diseases and severe hypersensitivities (2). In addition, Tregs play crucial role for graft tolerance and survival (3), while their higher numbers in allogeneic hematopoietic stem cell grafts predict improved survival of the recipient after transplantation (4). Recently Tregs were shown to be essential for resolving atherosclerotic cardiovascular disease, including atherosclerotic plaque regression (5)

Natural thymus derived $CD4^+FoxP3^+$ Tregs present a large therapeutic potential. They inhibit inflammatory responses, ameliorate autoimmunity and at the same time do not impair physiological immune responses against pathogens, like known immunosuppressive drugs (6). Multiple cell populations are under Treg surveillance, including conventional T cells, B cells, monocytes/macrophages, dendritic cells (DCs), granulocytes, natural killer (NK) cells and mast cells. Therefore, Tregs are responsible for establishment and maintenance of peripheral tolerance (1), as well as tissue protection and regeneration (7). To fulfil this duty Tregs use a wide variety of mechanisms of action. However the most universal and crucial are: 1). suppression of target cell proliferation and 2). degradation of extracellular adenosine triphosphate (eATP), extracellular adenosine diphosphate (eADP) and extracellular adenosine monophosphate (eAMP) that leads to generation and accumulation of extracellular adenosine (eADO). Suppression of immune cell proliferation significantly ameliorates any immune response and enables the shift into immune tolerance. To achieve this goal and inhibit proliferation of conventional T cells, like effector T cells (Teffs) Tregs use a mechanism called interleukin 2 (IL-2) deprivation (8). IL-2 is a T cell mitogen. After antigen recognition activated T cells start to secrete IL-2 that acts in autocrine and paracrine manner, inducing their proliferation. Unlike other T cells, Tregs do not produce IL-2, but the cytokine is required for their function and survival. Therefore, Tregs are characterized by increased expression of IL-2 receptor α-chain (IL2Rα/CD25) that together with CD122 (IL2Rβ) and CD132 (IL2Rγ) forms trimeric receptor which binds IL-2 with significantly higher-affinity, than dimeric IL2 receptor (IL2R) composed of CD122 and CD132 expressed by conventional T cells (9).

Another very universal mechanism of suppression of immune responses by Tregs is based on eATP degradation leading to generation of extracellular adenosine (eADO). Following tissue injury high amounts of ATP are released into extracellular space. When eATP binds to P2 receptors (virtually expressed on all immune cells) it induces cell activation and inflammatory responses (10). Therefore, inhibition of eATP-gated P2X receptors has been shown to halt lymphocyte activation, suppress both Th1 and Th17 cells and to promote Treg responses. Not surprisingly, the inhibition of eATP signalling was found to attenuate GVHD in animal model, downregulate alloimmune responses, expand Tregs and promote graft survival (11). eATP signalling can be limited by internal regulatory mechanisms that redirect immune response from activation to suppression leading to generation of eADO. In contrary to eATP, eADO binds to P1 receptors and exerts immunosuppressive effects (10). In canonical pathway adenosine is produced through the action of adenosinergic ectoenzymes expressed on the cell membrane: CD39- triphosphate diphosphohydrolase (NTPDase) 1 and CD73-5'-nucleotidase (5'-NT). CD39 converts ATP into ADP and ADP into AMP, while CD73 dephosphorylates the latter to eADO (12). Both CD39 and CD73 are expressed by Tregs and are known to be crucial for their immunoregulatory function (1). It has been also reported recently that Treg expression of CD39 and its high potential for eATP degradation is crucial for development of operational tolerance after renal transplantation (13).

Mounting evidence has linked alterations in the generation of eADO from extracellular nucleotides by ectonucleotidases to the immunological disruption and loss of immune tolerance in autoimmunity (10). As eADO suppresses function and inhibits proliferation of various populations and subsets of immune cells, including T and B lymphocytes, NK cells, DCs, monocytes, and macrophages, stimulates release of anti-inflammatory cytokines (i.e., IL-10) and inhibits the release of proinflammatory mediators (i.e., tumour necrosis factor α; TNF-α and nitric oxide) it plays a significant role in restoring immune homeostasis, preventing tissue damage and promotion of healing processes (14,12). Multiple reports indicate that the onset of autoimmune disorders is-at least partially-related to a disruption of the adenosinergic pathways and to a local defective production of eADO. Multiple studies reported the link between eADO production and pathogenesis and/or clinical outcome of multiple sclerosis (MS) (15-16), rheumatoid arthritis (RA) (17), juvenile idiopathic arthritis (JIA) (18), autoimmune uveitis (AU) (19), type 1 diabetes (T1DM) (20), renal diseases (21) and GVHD (22). In in the latest eADO was shown to selectively deplete alloreactive CD4 and CD8 T cells, preventing onset of GVHD, but preserving immunity to viruses and leukaemia (23).

Therefore, providing of in vitro expanded Tregs that not only express CD39 and CD73, but first of all, present robust activity of these surface enzymes leading to eADO accumulation is crucial for Treg based therapies. Such Tregs present robust functionality and their clinical use will be of the great therapeutic benefit. What sets the goal of the invention.

The authors of the presented invention has been focused on the method of in vitro production of functionally potentiated Tregs . The team has been using Tregs for clinical therapies since 2008 and was the first who applied in vitro expanded Tregs for the treatment of GVHD in adults (NKEBN/458-310/2008) (24), then T1DM in children (TregVAC ISRCTN06128462; TregVAC2.0EudraCT:2014-004319-35) (25), and subsequently MS (TregSM EudraCT:2014-004320-22) (26).

Currently, clinical trials that test therapeutic potential of Tregs have been conducted in multiple medical centres all over the world. In these studies Tregs are used for therapy and/or prevention of GVHD (e.g. Treg002EudraCT:2012-002685-12) (27), treatment of T1DM in adults (NCT01210664), therapy of amyotrophic lateral sclerosis (ALS; NCT04055623), tolerance induction in kidney (e.g. NCT02091232 and NCT02129881) and liver (ThRIL NCT02166177 and NCT01624077) recipients (26). Results of all these studies confirm that Treg treatment is safe and does not impair immune responses to foreign and dangerous antigens (e.g. viruses, bacteria, tumour cells) (6). However, therapeutic use of Tregs requires their isolation and ex vivo expansion before clinically relevant numbers are obtained. Therefore, inventors have work on Treg based therapies and have been struggling with the same technical problems associated with Treg isolation and culture. The first of these issues was the low purity of isolated population, that affected safety and efficacy of the treatment (24). The hurdle was solved by introduction of a cell sorter for Treg isolation, as described in EP2859092A1. Nevertheless, ex vivo Treg expansion is associated with significant loss of their immunosuppressive potential that directly diminishes efficacy of Treg based therapies (28).

In addition, Tregs derived from patients with pathologically altered immune responses (e.g. autoimmune diseases, allergies, GvHD) are characterized by impaired functionality (29). Therefore, Treg production for clinical therapy should optimally not only preserve their function during culture in vitro, but also enhance their therapeutic potential.

Currently, the most common method for Treg expansion is based on use of IL-2 and magnetic beads coated with anti-CD3 and anti-CD28 antibodies (CD3/CD28 beads) (30-31). However, multiple groups reported that this method does not prevent loss of Treg phenotype and function over the time in vitro (28). Therefore, numerous research groups used to add rapamycin into the culture, as it was shown to keep high numbers of CD4$^+$FoxP3$^+$ Tregs during expansion (32). However, recent studies confirm that the drug preferentially inhibits proliferation of conventional T cells (Teffs) that can contaminate Treg culture, but does not prevent loss of FoxP3 expression in natural Tregs during the second week of expansion (33). Noteworthy, presence of rapamycin was shown to be associated with 5-10-fold reduction in Treg proliferation (34). Landman S et al. used 5-aza-2'-deoxycytidine (DAC) a derivative of 5-Azacytidine which is therapeutically used in haematological malignancies and is DNA methyltransferase inhibitor (DNMTi). However, the group reported that in vitro activation of Tregs in the presence of DAC led to a significant inhibition of Treg proliferation. In addition, Treg activation in the presence of DAC led to increased interferon γ (IFNγ) expression and induction of a T helper-1 phenotype (Th1) (35). Another approach that has been tested in Treg culture also by our group was based on use of infliximab, an anti-TNFa (tumour necrosis factor α) antibody. We have found that presence of the drug in the culture medium preserved function of Tregs and their characteristic phenotype over time (36). In a different study we have indicated that mild hypothermia of 33°C is able to stimulate Treg proliferation and preserve their phenotype (31). Multiple groups have been tested the impact of retinoic acid on Tregs and reported that the compound sustained Treg stability under inflammatory conditions (37). However, all these approaches are distinct from the current invention.

The main goal of our invention was to condition Tregs to obtain modified Treg population in vitro with stronger therapeutic potential. Unexpectedly the goal was achieved by implementation of adipose tissue derived mesenchymal stem cells (ASCs) from nonrelevant allogeneic donors into Treg culture. However, according to the current invention to avoid ASCs transfer into Treg recipient both cell types are separated from each other with a cell impermeant membrane. Nevertheless, the cells communicate with each other with soluble mediators and due to this indirect interaction both cell types modify their environment bilaterally. The unique cytokine cocktail secreted into the culture medium by Tregs and allogeneic ASCs conditions (modifies) Tregs, thus they demonstrate stronger therapeutic potential. This way modified Treg population according to the invention shows enhanced suppression of proliferation of conventional T cells (Teffs) and enhances accumulation of eADO known of its pro-tolerogenic and anti-inflammatory potential.

Patent application WO 2010/135255 A1 disclosed a method for ex vivo expansion of CD4$^+$CD25$^+$ Tregs during 3 weeks. In addition, the immunomagnetic methods of Treg isolation described in this patent resulted in low initial Treg purity (40-75% on day 0), thus consequently a percentage of FoxP3$^+$ cells in the second week of the culture on average was equal to 40-50 %. The method of Treg production described in our patent application significantly differs. It is based on use of combination of immunomagnetic isolation and cell sorting with FACS (florescence activated cell sorter). This difference enables isolation of Tregs with median purity of 98%, thus post expansion purity is significantly higher (median % of CD4+FoxP3+ Tregs =85%), than it was described in the invention no. WO 2010/135255 A1. However, the main novelty of the current application is Treg coculture in presence of membrane separated ASCs that results in significant enhancement of Treg therapeutic potential.

Patent application US 2015/0104428 A1 describes immunoregulatory effect of bone marrow derived mesenchymal stem cells (MSCs). The authors describe that MSCs after systemic infusion induce apoptosis of conventional T cells and increase Treg numbers in peripheral blood. This approach significantly differs from our invention too. In our approach mesenchymal stem cells are not isolated from bone marrow, but from adipose tissue (thus they are called ASCs) and are not supposed to be administered to the patients for induction of Treg responses. In our approach ASCs are used for enhancement of Treg activity in vitro before the latest will be used for therapy.

In the previous studies MSCs, including ASCs were used to induce immune suppression/tolerance by their direct administration to the patients (38), but not via stimulation of Treg function in vitro. Previously, the effect of MSCs on Tregs was demonstrated only in vivo. In addition there is lack of studies were MSCs or ASCs were cocultured with pure sorted Treg subset in direct, as well as in indirect contact.

Elaboration of a safe, simple to implement and economic method for expansion of stable Tregs characterised with potent suppressor activity is of key importance for advancement and success of clinical trials utilising Tregs as a therapeutic tool. Ability to regulate the immune response is important from the therapeutic point of view. Physiologically, the immune system recognises and destroys foreign and dangerous antigens, and tolerates self-tissues. Nevertheless, in autoimmune diseases, such as MS, T1DM, psoriasis, systemic lupus erythematosus (SLE) or RA this mechanism is impaired (25-26,39-42) and immune system begins to destroy the patient's own tissues, leading to irreversible changes. The current management of autoimmune diseases commonly involves nonantigen-specific immunosuppression and inhibition of inflammatory response. However, this kind of treatment emerges ineffective in long time perspective. Despite the initial improvement, progression of the disease cannot be completely stopped, while discontinuation of the treatment usually results in an exacerbation of the symptoms. Moreover, this treatment is associated with compromised immunity (43). Therefore, the patients become susceptible to various infections, which have much more severe manifestation, than in healthy individuals who are not treated with immunosuppressive drugs. Furthermore, non-specific immunosuppression increases the risk of cancer (higher prevalence of cancer in patients receiving immunosuppressants) (44).

Regulation of the immune response is also an important issue from the viewpoint of transplantation medicine. Organ transplantation is usually a life-saving procedure but is also associated with life-long treatment with potent immunosuppressive drugs. Discontinuation of this treatment results in increased immune response to the graft, which leads to its loss. The use of immunosuppressive drugs, as in therapy of autoimmune diseases, is associated with the risk of severe adverse effects. Furthermore, some of these drugs simultaneously protect the transplanted organ from the recipient immune system and are toxic for the graft or other tissues. Examples include nephrotoxic inhibitors of calcineurin (ciclosporin and tacrolimus) used in kidney transplantation (45) or rapamycin used in pancreatic islet recipients despite its deleterious impact on transplanted $\beta$ cell function (46).

The problem of immunosuppression and regulation the of immune response is also closely associated with bone marrow transplantation. The principal difference between organ and bone marrow transplantation is that in the former case, medicine intends to protect the transplanted organ from the destructive action of the recipient's immune system, while in the latter case, there is no risk of transplant rejection but the transplanted bone marrow is a source of cells that attack the recipient's body and may lead to death (47). However, regardless of the origin of the immune cells attacking the patient's body, currently the fight against the excessive immune response is based on using of non-specific immunosuppression.

An apparently different issue is the excessive immune response to innocuous foreign antigens, as it is observed in various types of hypersensitivities that all together are used to be called allergies. Most of these disorders can be successfully treated symptomatically with the available medications. Nevertheless, in case of asthma the problem is more complex. The disease progresses with time, inflammation exaggerates leading to persistent structural changes in the airways and medical treatment become ineffective (48). Thus, there is need for novel therapeutic approaches.

To summarize, fully effective and safe drugs used for treatment of autoimmune and allergic diseases, as well as in transplant recipients would be those that target directly the mechanisms responsible for the disease onset or injury of the transplanted organ and at the same time would not impair the physiological immune response to foreign and dangerous antigens. A chance for so-called intelligent immunosuppression is offered by Tregs. However, the success of clinical therapy with Tregs depends on the functionality of Treg population and method for Treg production,, that will guarantee safety and efficacy of the treatment (49).

Thus goal of the invention was to provide the effective Treg population with better functionality and elaborate the method for production of polyclonal, as well as antigen-specific Tregs with enhanced immunoregulatory potential (functionality) suitable for clinical therapy and research approaches.

Unexpectedly, according to performed studies, authors of the current invention have demonstrated potentiation of Treg function by the culture in indirect contact with ASCs derived from allogeneic HLA donors. The method enables to achieve higher numbers of Tregs characterized by significantly enhanced immunosuppressive activity - functionality - when ASCs from nonrelevant donor are added into the same culture with Tregs. The main novelty of the invention is that Tregs and ASCs are separated from each other with a cell impermeant membrane with pore size ≤0.4 $\mu$M. during the process of cell production. The membrane separates the two cell types physically, preventing cell-to-cell contacts and cellular migration between the culture compartments. Preferably pore diameter of the mesh is equal or lower than

0.4 μm. For example the permeable membrane is made out of translucent high density polyethylene terephthalate (PET) and have the pore size mentioned above. However, at the same time the membrane enables the unrestrained passage of soluble mediators between the two culture compartments (above and beneath the membrane). In the example, sterile cell culture inserts with low protein binding PET membranes with a smooth surface and defined cylindrical pores that traverse the membrane were used. There are available multiple types of culture inserts with various pore sizes, but according to the invention the optimal pore size is of 0.4 μm or lower.

Thus, according to the invention, Tregs and ASCs modify their environment bilaterally and the culture method permits indirect communication between the cells. As a result, the unique cytokine cocktail is secreted into the culture medium by Tregs and allogeneic ASCs. Subsequently this cytokine cocktail conditions (modifies) Tregs, thus they demonstrate stronger therapeutic potential and more robust proliferation, than Tregs cultured in standard (known) monocultures. The described Treg population is therefore new and it is characterized by enhanced immunosuppressive potential (functionality).

The method according to the invention enables production of conditioned Tregs - the Treg population with enhanced functionality that are characterized by several advantages:

Enhanced proliferation- during 7 day expansion the median number of Tregs collected from the coculture is 18% higher, than it is observed for Tregs expanded in standard culture conditions (monoculture).

Higher proportion of CD25$^{High}$ Tregs at day 14 in vitro (median 91.7%, range 85.3%-98.6%) as compared with standard culture conditions (monoculture; median 82.1%; range 58.1%-92.6%).

Higher proportion of CD73$^{+}$ Tregs at day 14 in vitro (median 3.15%; range 1.6-8.6%) as compared with standard culture conditions (monoculture; median 1.8%, range 0.8-2.7%).

Higher expression (MFI, median fluorescence intensity) of CD73 by CD73$^{+}$ Tregs at day 14 in vitro; MFI of CD73 of Tregs from the coculture is 30% higher, than corresponding MFI of Tregs expanded in standard culture conditions (monoculture).

Enhanced inhibition of proliferation of conventional CD4$^{+}$ T cells (effector T cells, Teffs); the conditioned Tregs are significantly more potent inhibitors of proliferation of Teffs, than Tregs expanded in standard culture conditions (monoculture). Proliferation index of Teffs in presence of the conditioned Tregs is 11%, 14.54% and 23.12% (median) lower for 2:1, 1:1 and 1:2 Tregs:Teffs ratios, respectively as compared with the values observed for Tregs expanded in standard culture conditions (monoculture).

Enhanced degradation of eATP; 1 x10$^{6}$ of conditioned Tregs degrades 26% (median) more nmol of eATP during 1 min, than Tregs expanded in standard culture conditions (monoculture).

Enhanced degradation of eAMP; 1 x10$^{6}$ of conditioned Tregs degrades 244.4 % (median) more nmol of eAMP during 1 min, than Tregs expanded in standard culture conditions (monoculture). Degradation of eAMP leads directly to generation of eADO that is known immunosuppressive and protolerogenic mediator.

The main problem in Treg culture that has been reported in the state of the art is their progressive loss of immunosuppressive function during culture in vitro and low proliferative potential. However, the current invention by implementation of ASCs into Treg culture enables to obtain ≈20% more Tregs after 7-day expansion. Number of proliferating cells grows exponentially. Therefore, the difference in the final cell count between the culture conditions will increase proportionally to time of culture duration. Tregs expanded according to the invention are also characterized by significantly higher proportion of cells expressing CD25, an indispensable subunit of high affinity IL2R. This indicates that the Tregs expanded in indirect contact with ASCs can bind significantly more IL-2, than Tregs expanded in monoculture and thus show stronger potential for inhibition of proliferation and survival of conventional T cells via IL-2 deprivation mechanism.

Consequently, the invention allows to decrease the duration of Treg expansion and thus to be used for clinical treatment of more patients within the same time, concomitantly preserving higher quality and stability of the expanded cells. The high proliferative potential of Tregs expanded in indirect contact with ASCs is at least partially associated with a higher and more stable expression of CD25 molecule on the surface of these cells (a higher % of CD25$^{High}$ Tregs in the coculture). As it was mentioned before CD25 is a subunit of high affinity receptor for IL-2, a cytokine that is a mitogen of all T cells and is essential for generation, proliferation and function of T cells including Tregs.

The most important advantage of the invention are Tregs with significantly higher immunosuppressive potential than it is observed when the cells are expanded in accordance with the conventional known methods (monocultures). The provided example indicates that Tregs expanded in indirect contact with ASCs are able to substantially suppress proliferation of conventional T cells (Teffs). Tregs conditioned with ASCs according to the method disclosed herein almost completely suppressed proliferation of target cells in the studied 2:1, 1:1 and 1:2 Treg: Teff ratios. At the same time both: 1). Tregs expanded alone in basic culture medium, as well as 2). Tregs cultured in medium supplemented with culture supernatants (SNs) collected from ASC monocultures were significantly less effective suppressors of Teff proliferation. This indicates that the culture protocol disclosed herein is a novel and very effective method of " Treg conditioning" that have very high therapeutic potential.

Previously, a demethylating agent 5-aza-2' -deoxycytidine (DAC) was tested in Treg culture as the drug that can potentially preserve Treg phenotype and function. However, the compound was shown to suppress cell proliferation and induce expression of proinflammatory IFN$\gamma$ that can affect therapeutic potential of Tregs. While the current invention enables to overcome this problem, as Treg culture in indirect contact with ASCs stimulates Treg proliferation, does not impair viability and significantly improves their therapeutic potential. Tregs expanded in presence of membrane separated ASCs show enhanced potential for degradation of eATP and eAMP, and thus increased generation of immunosuppressive eADO. At the same time indirect contact of Tregs with ASCs does not increase eADO conversion to inosine (INO), leading to its extracellular accumulation. As it was mentioned before eADO is one of the most important inducers of immune tolerance.

It is known that Tregs use CD39 and CD73 ectonucleotidases for degradation of ATP and AMP, respectively. Tregs expanded according to the method disclosed herein show increased activity of both CD39 and CD73 ectonucleotidases. In addition, the inventors showed and observed a negative correlation (R=-0.48; p=$3 \times 10^{-3}$) between Treg degradation of eAMP leading to accumulation of eADO and proliferation of Teffs in presence of these Tregs. The data indicate that the higher Treg potential for eADO accumulation is, the stronger suppression for Teff proliferation they demonstrate. Thus, the basis of invention is that the therapeutic potential of Tregs can be enhanced significantly by conditioning them by indirect contact with ASCs.

In addition, separation of ASCs from Tregs with the membrane increases safety of this culture protocol. Unlike in cocultures where cells are expanded in direct cell-to-cell contact in the same culture vessel (plate, flask or sack), our model prevents transfer of allogeneic ASCs into Treg recipient, as the cells do not contact directly. Thus this kind of approach protects the recipient from delivery of allogeneic ASCs that might generate adverse effects.

The present invention relates to the Treg cell population and method of in vitro expansion of CD4$^+$ FoxP3$^+$ Tregs. According to the invention, Treg expansion takes place in indirect contact with allogeneic human ASCs which are separated from each other with membrane that prevents their direct cell-to-cell contact but does not block exchange of secreted soluble mediators between the separated cells. The process of Treg expansion takes place permanently or temporarily but at least 3 days (72 hours) in presence of membrane separated ASCs. Temporarily means that the Tregs and ASCs can be cultured separately and then coculture is performed or are cocultured together and then are separated into monocultures.

The advantages of the invention are presented in following figures and table.

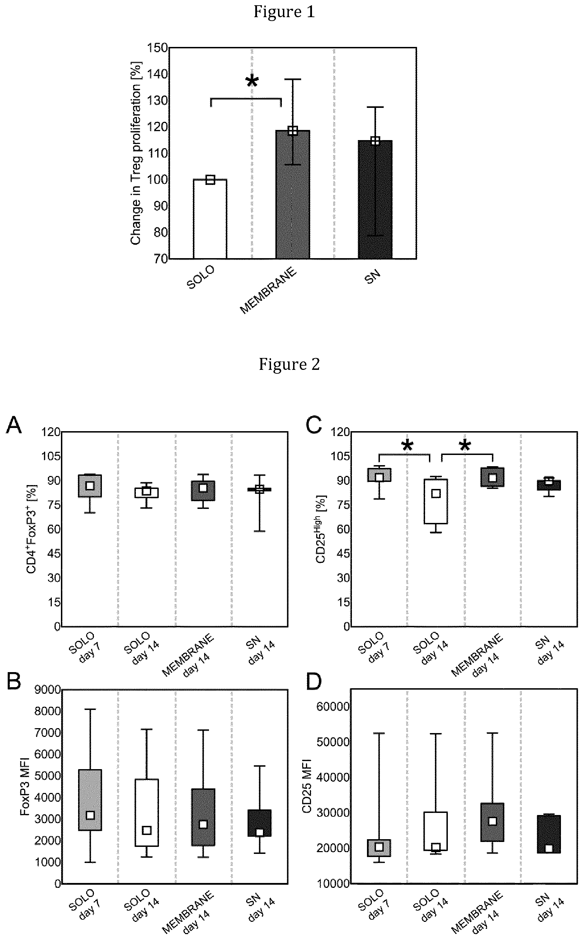

Figure 1 is a chart depicting changes in Treg numbers in cultures with membrane separated ASCs and the cultures in medium supplemented with ASC derived SNs in relation to Tregs expanded alone in basic culture medium. The number of Tregs expanded in standard monoculture was treated as a reference and set to 100% for each experiment. Thus, values for the other tested conditions are showed as % of values reached in the standard cultures.

Figure 2 is a chart showing % of FoxP3$^+$ and CD25$^{High}$ cells during expansion at tested culture conditions and intensity (median fluorescence intensity, MFI) of these markers expression by FoxP3$^+$ and CD25$^{High}$ cells, respectively.

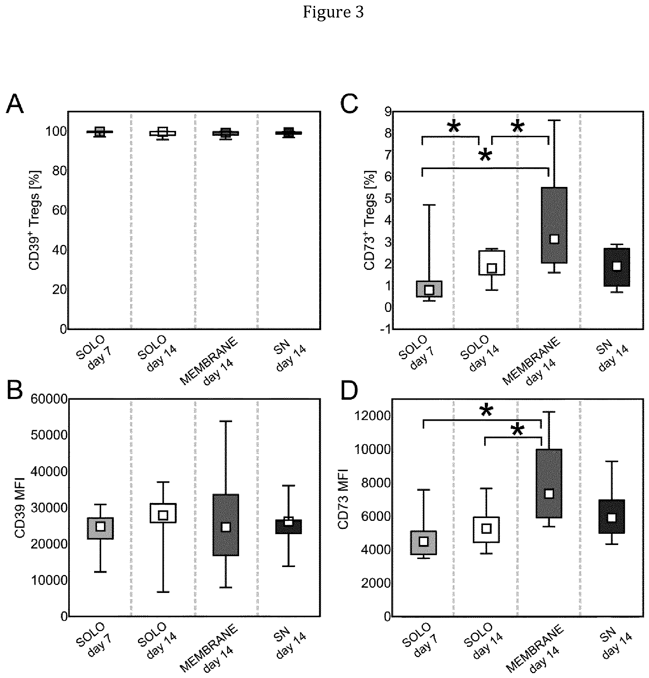

Figure 3 is a chart showing % of CD39$^+$ and CD73$^+$ Tregs during expansion at tested culture conditions and intensity (MFI) of these markers expression by CD39$^+$ and CD73$^+$ cells, respectively.

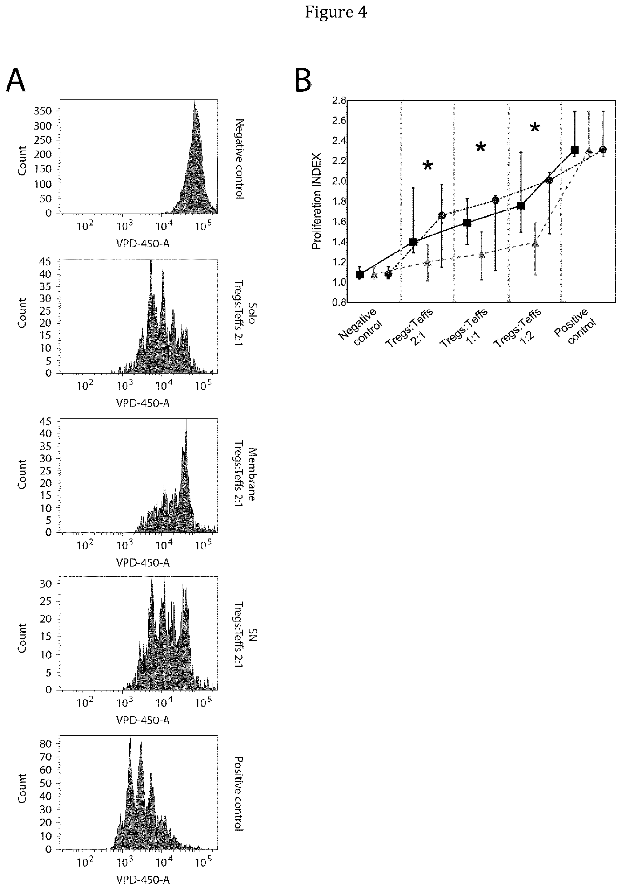

Figure 4 is a chart showing representative histograms of Teff proliferation inhibition by Tregs expanded in tested culture conditions in 2:1 Treg:Teff ratio and depicts proliferation index (PI) of Teffs stimulated with CD3/CD28 beads in presence of Tregs previously expanded alone in basic culture medium, in culture medium supplemented with ASC derived SNs and in presence of membrane separated ASCs in 2:1, 1:1 and 1:2 Treg:Teff ratios. For comparison the data for unstimulated autologous Teffs and bead stimulated Teffs cultured alone are presented as negative and positive controls, respectively.

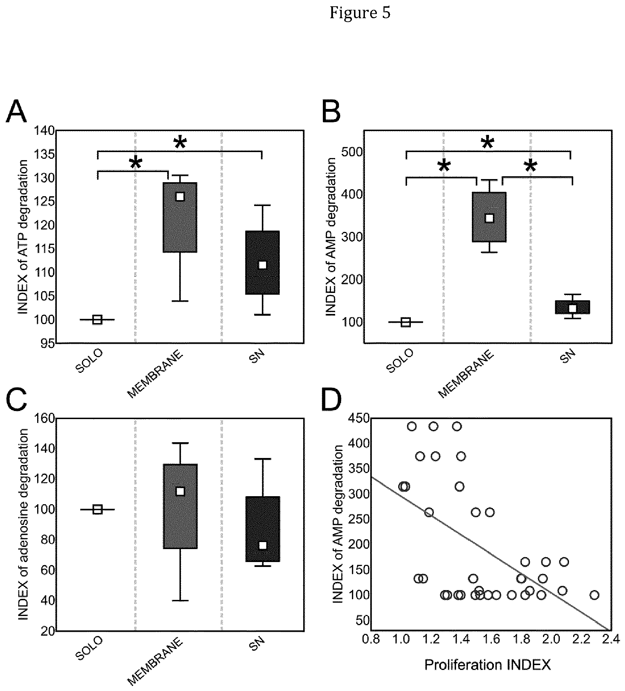

Figure 5 is a chart showing degradation of extracellular ATP, AMP and adenosine by Tregs previously expanded in tested culture conditions. The data are shown in % as indexes. The fig. also presents correlation between AMP degradation into adenosine and suppression of Teff proliferation in proliferation inhibition tests. Proliferation of Teffs is presented as proliferation index.

Table 1 is a table showing levels of 51 cytokines measured in supernatants collected after 24h and 48h from Treg monocultures, cocultures of Tregs and ASCs separated with cell impermeant membranes and from ASC monocultures.

The present invention is illustrated by the following example

Example

Isolation of Tregs and CD4$^+$ Teffs

Peripheral blood Tregs and CD4$^+$ Teffs were isolated from buffy coats derived from female and male volunteer blood donors (n= 9) admitted to Regional Centre for Blood Donation and Treatment in Gdansk. First, peripheral blood

mononuclear cells (PBMC) were obtained by Ficoll/Uropoline gradient centrifugation. Then, CD4$^+$ T cells were isolated with negative immunomagnetic selection (isolation purity 90-99%). For this purpose, the EasySep Human CD4+ T Cell Enrichment Kit (Stemcell Technologies) was used. The kit depletes the samples from cells expressing the following antigens: CD8, CD14, CD16, CD19, CD20, CD36, CD56, CD66b, CD123, TCR$\gamma/\delta$ and glycophorin A. Subsequently, the isolated CD4$^+$ T cells were labeled with monoclonal antibodies (mAb, 5$\mu$l mAb/10$^6$ cells) specific for the following antigens: CD3, CD4, CD25, CD127, CD8, CD19, CD16 and CD14 (BD Biosciences). The last 4 mAbs were conjugated with the same fluorochrome in aim to minimize the fluorescence overlap and cut-off in one step cytotoxic T cells (Tc), B cells, NK cells and monocytes, respectively. These cells were defined all together in sorting algorithm as lineage.

Then, the cells were sorted with florescence activated cells sorter (FACS; Aria II, BD Biosciences) into the following phenotype of Tregs: CD3$^+$CD4$^+$CD25$^{High}$CD127$^{-/Low}$doublet$^-$lineage$^-$ and Teffs: CD3$^+$CD4$^+$CD25$^-$CD127$^{High}$doublet$^-$lineage$^-$. The post-sort purity of Tregs was ~100% [median(min-max): 98%(97-99)]. The method for Treg culture according to the present invention allows to replicate the results of Treg expansion also when different model of cell sorter or Treg isolation methods are used. Therefore, neither method of Treg isolation, nor the cell sorter used in this example are limitations of the present invention.

The isolated Tregs and Teffs were then seeded into separate plates and expanded at 37°C in X-vivo20 culture medium (Lonza) which meets GMP criteria. The medium was also supplemented with antibiotics (penicillin 100 U/ml; streptomycin 100 mg/ml), 10 % foetal calf serum (FBS) and interleukin-2 (IL-2; 2000 U/ml; Proleukin; Chiron, San Diego, CA). On the day 0 of the culture, magnetic beads coated with anti-CD3 and anti-CD28 antibodies (CD3/CD28 beads; Invitrogen; Carlsbad, CA) were added to the culture at a 1:0.6 cell: bead ratio. The beads mimic stimulation by antigen-presenting cells (APCs) and thus induce Treg proliferation. For the first 7 days Tregs were expanded in monocultures, while Teffs were expanded alone for the entire duration of the experiment (14 days). Teffs served as a control for Treg phenotype analysis and were used as target cells in proliferation tests. The type of culture medium, serum and CD3/CD28 beads are not the limitations of the current invention and can be replaced by other equivalent reagents known in the art and used for standard T cell culture.

Isolation of ASCs

ASCs were isolated from fresh samples of adipose tissue (50-200 ml) obtained during liposuction procedures from 9 healthy individuals who were unrelated with Treg and Teff donors. Then, samples were washed 3 times with PBS to remove contaminating debris and erythrocytes. Subsequently, the tissue was digested with digestion solution in 1:1 of digestion solution (ml): tissue mass (g) ratio at 37°C with gentle agitation to obtain single cell suspension. Composition of the digestion solution was as follows: 5% collagenase type I (Sigma-Aldrich; 5mg/ml), 19% of PBS and 1% of FBS. The collagenase was inactivated with an equal volume of LG-DMEM (PAA, Austria) containing 10% FBS, followed by filtration of the resulting cell suspension through a 100 $\mu$m nylon cell strainer (Falcon) in order to remove debris. The filtrate was centrifuged and washed with PBS. Then, the pellet was suspended in erythrocyte lysis buffer for 10 min. (at room temperature, RT) to remove erythrocytes. Subsequently, the cells were centrifuged (600g, 10 min.) and washed with PBS containing 4% FBS. Isolated cells represented an initial stromal vascular cell fraction (SVF) and were seeded at a density of 1 x 10$^5$ cells/cm$^2$ into 25cm$^2$ flasks with vented caps (Corning Primaria) in LG-DMEM containing 10% FBS (37°C; 5% CO$_2$). Following the first 5 days of initial plating, nonadherent cells were removed by intense washing and the remaining fibroblast-like adherent cells were maintained in LG-DMEM 10% FBS medium until they achieved 80-90% confluence (~7 days of initial cultivation).

Subsequently, the cells were harvested after trypsinization (0.025% trypsin/EDTA, Lonza) at 37°C for ≈3 min. Then, their potential for differentiation into osteoblasts, adipocytes, and chondroblasts under standard in vitro differentiation conditions was tested to confirm stem cell properties. Simultaneously, the remaining ASCs were seeded at density of ~ 7x10$^3$ cells/cm$^2$ into 75cm$^2$ flasks (Corning Primaria) and cultured for further experiments.

Treg coculture with ASCs

After reaching ~ 80% confluency ASCs in 2nd passage were harvested and analyzed with flow cytometry (LSRFortessa, BD Biosciences) to confirm a phenotype of mesenchymal stem cells. Only cultures where ≥95% of cells presented the following phenotype: CD105$^+$CD90$^+$CD73$^+$CD44$^+$CD45$^-$HLA-DR$^-$ were planned to be used for further experiments. However, the required purity was reached each time and all isolated ASCs passed the quality control. Subsequently, ASCs and Tregs were seeded into 24-well plates (Corning Primaria; at 3:20 ASC:Treg ratio in 1000 $\mu$l of medium/well) in the following combinations:

1). Tregs Solo (2x10$^5$ cells/well)- control monoculture of Tregs.
2). ASCs Solo (3x10$^4$cells/well)- control monoculture of ASCs.
3). ASCs SNs (3x10$^4$cells/well)- monoculture of ASCs from which supernatant (SN) was collected daily and transferred into Treg culture (Tregs SNs).

4). Tregs SNs ($2 \times 10^5$ cells/well)- Treg monoculture supplemented with SNs derived from ASCs (ASCs SNs).

5). Membrane- coculture of Tregs ($2 \times 10^5$ cells/well) and ASCs ($3 \times 10^4$ cells/well) separated from each other with a high density polyethylene terephthalate (PET) membrane. The membrane separating the two cell types was a mesh with apertures with diameter of 0.4 $\mu$m. Therefore, it prevented cellular migration through the mesh and thus the direct cell-to-cell contacts. However, the membrane allowed exchange of the soluble mediators and receptors secreted by the two cell types into the culture medium. ASCs were seeded in culture plate, while Tregs were added to the same culture wells but within culture inserts with aforementioned membrane (pore size of 0.4 $\mu$m) suspended above ASCs. Thus, secretion of biologically active molecules by one cell type affected responses of the second cell type and vice versa. However, there was a lack of physical contact between the two cell types and no exchange of extracellular vesicles (EVs) with diameter of >0.4 $\mu$m occurred.

In these experiments all cells were expanded in X-vivo20 (Lonza) medium supplemented with antibiotics, 10% FBS, IL-2 (2000 U/ml) and CD3/CD28 magnetic beads in 1:0.6 Treg:bead ratio. Each time ASCs in 3rd passage were used in these experiments. In case of condition no. 4 (Tregs SNs) every day 800 $\mu$l (80%) of the culture medium was removed without the cells and replaced with 800 $\mu$l of the medium collected from ASCs cultured in condition no. 3 (ASCs SNs). Then, fresh medium was added to the condition no. 3 to keep the volume of 1000 $\mu$l/ well. In addition, every 24h and 48h culture supernatants were collected from condition no. 1, 2 and 5 to compare cytokine secretion by Treg and ASC monocultures and membrane separated coculture.

After 7 day expansion Tres and ASCs were collected counted, analyzed with flow cytometry and subjected for functional tests (proliferation test, analysis of eATP, eAMP and eADO degradation).

Tregs counting after coculture

After 7 days, the Tregs were harvested and washed with a PBS. Beads were removed with a magnet and the cells were counted. Subsequently, the number of Tregs expanded in monoculture (standard culture protocol) was treated as 100% and the differences between this and the experimental culture conditions 4 and 5 were calculated and presented in %. It was observed that 7-day culture of Tregs in presence of membrane separated ASCs (n=9; MEMBRANE) resulted in ≈20% (median) higher cell counts as compared with Tregs from the standard monocultures (n=9; SOLO; Mann-Whitney U test, p=$1 \times 10^{-4}$). Treg culture supplementation with ASC derived SNs (n=9; SN) was less effective stimulator of Treg proliferation, than coculture in presence of membrane separated ASCs (Fig. 1).

Phenotype control

On each 7th and 14th day after Treg isolation (before and after coculture with ASCs), samples of Tregs expanded in 3 tested conditions: 1). monocultures, 2). cocultures with membrane separated ASCs and 3). in medium supplemented with SNs derived from ASC monocultures were collected and labelled with monoclonal antibodies purchased from eBiosciences (unless otherwise stated) directed against the following antigens: CD4 (BD Biosciences), CD25 (BD Biosciences), CD127 (BD Biosciences), CD45RA (BD Biosciences), CD62L, Helios, FoxP3, HSP-60 (Abcam), HSP-70 (Abcam), HSP-90 (Abcam), CD39, CD73, CTLA-4, CD31 and Nrp-1 (neuropilin 1). Each time unstained cells served as negative control. All samples were treated with Foxp3 Staining Buffer Set (eBiosciences) to enable intracellular staining. Then, the cells were analysed with flow cytometer (LSRFortessa, BD Biosciences).

Tregs expanded in all tested conditions kept high expression of FoxP3 and no significant differences were found between the cultures in frequencies of FoxP3$^+$ T cells and intensity of FoxP3 expression by FoxP3$^+$ cells (Fig.2 A-B).

However, Tregs expanded in presence of membrane separated ASCs (MEMBANE day 14) showed significantly higher % of CD25$^{High}$ cells at day 14 after the isolation (CD25/IL2R$\alpha$; median 91.7%, range 85.3-98.6%, n=9) than those cultured in standard conditions (monoculture, SOLO day 14, n=9, Mann-Whitney U test, p=0.03; median 82.1%; range 58.1-92.6%). Differences in % of CD25$^{High}$ cells between Tregs expanded in medium supplemented with SNs derived (SN day 14, n=9, median 89.9%, range 80.2-91.6%) from ASC monocultures and Tregs from standard monocultures did not reach statistical significance. In addition, only Tregs expanded in presence of membrane separated ASCs showed stable frequency of CD25$^{High}$ cells (Fig. 2 C) and tendency towards increasing intensity of CD25 expression (MFI) between day 7 and 14 of the culture (Fig.2D; Mann-Whitney U test, p=0.11). High expression of CD25 is crucial for regulatory activity of Tregs. Capture of IL-2 via CD25 limits its availability for other immune cells and thus prevents their proliferation.

No differences were found between tested Treg cultures in terms of expression of such Treg markers as CD39 (Fig.3 A-B), Helios, CTLA-4 CD127, CD45RA, CD62L, HSP-60, HSP-70, HSP-90, CD31 and Nrp-1. In addition, expression of Helios (a marker of thymus derived Tregs) and CTLA-4 (an immunoregulatory molecule) was high and stable in all culture conditions. Median frequency of Helios$^+$ and CTLA-4$^+$ Tregs was equal to 85.5 and 100%, 82.5 and 100% and 80.1 and 100% for Tregs expanded in monocultures, in presence of membrane separated ASCs and those from monocultures supplemented with ASC derived SNs, respectively. Nevertheless, Tregs expanded in presence of membrane separated ASCs (MEMBRANE day 14, n=9) showed higher frequency of CD73$^+$ cells at day 14 after cell isolation (Fig.3 C; Mann-Whitney U test, p=0.02; median 3.15%; range 1.6-8.6%), than the cells cultured in standard conditions (SOLO day 14, n=9, median 1.8%, range 0.8-2.7%) and in medium supplemented with SNs collected from ASC monocultures (SN day 14, n=9, median 1.9%, range 0.7- 2.9%). In addition, CD73$^+$ cells from the cocultures concomitantly showed 30% higher expression of CD73 (MFI; Fig.3D; Mann-Whitney U test, p=0.01), than cells from the standard monocultures. Moreover, Tregs from the cocultures at day 14 after isolation were characterized by higher frequencies of CD73$^+$ cells (Mann-Whitney U test, p=1x10$^{-3}$) with higher expression of the molecule (MFI; Mann-Whitney U test, p=7x10$^{-3}$), than Tregs from 7-week monocultures (SOLO day 7). These data indicate that the expression of CD73 in Tregs increased in response to contact with membrane separated ASCs during the culture in vitro.

Test of inhibition of Teff proliferation by Tregs

At day 7 of the coculture a proliferation inhibition assay was performed. Teffs were stained with 2 $\mu$M Violet Proliferation Dye 450 (VPD-450; BD Horizon) for 15 min. at 37°C and mixed with unstained autologous Tregs derived from studied culture conditions in the following Treg:Teff ratios: 2:1, 1:1 and 1:2. Cells were cocultured for the next 4 days at 37°C in X-vivo20 medium supplemented with 10% FBS and CD3/CD28 beads in a 1:1 Teff:bead ratio. Simultaneously, VPD-450 stained and unstimulated Teffs were used as negative control (complete inhibition of Teff proliferation, n=6), while VPD-450 stained Teffs and stimulated with CD3/CD28 beads in 1:1 Teff:bead ratio with no Treg supplementation served as positive control (no Treg dependent inhibition, maximal proliferation of Tregs in the studied settings, n=6; Fig.4). After 4 days cells were collected and labeled with 7-aminoactinomycin D (7-AAD, BD Pharmingen) that binds to DNA of dead cells in aim to exclude dead cells from the analysis. Then the samples were evaluated with flow cytometry (LSRFortessa, BD Biosciences).

The data were analyzed as proliferation index (PI) that is an average number of divisions that all responding cells have undergone since the initiation of the culture and was calculated with the following formula:

$$PI= \frac{\text{The total number of divisions}}{\text{The number of cells that went into division}}$$

$$\text{The total number of divisions} = (n1/2)*1 + (n2/4)*2 + (n3/8)*3 + (n4/16)*4 + (n5/32)*5$$

n0    = number of cells that did not divide
n1    = number of cells that divided once
n2    = number of cells that divided twice
n3    = number of cells that divided 3 times
n4    = number of cells that divided 4 times
n5    = number of cells that divided 5 times

$$\text{The number of cells that went into division} =$$
$$= \text{The number of cells at start of the culture} - n0$$

$$\text{The number of cells at start of the culture} =$$
$$n0+ (n1/2) + (n2/4) + (n3/8) + (n4/16) + (n5/32)$$

Each time results for negative control correspond to the complete inhibition of Teff proliferation (lack of stimulation, Fig.4, Negative control), while results for the positive control correspond to proliferation of stimulated Teffs in absence of Tregs (lack of inhibition, Fig.4, Positive control). Tregs cultured in presence of membrane separated ASCs were found to suppress proliferation of Teffs (lower PI of Teffs, Fig. 4A histogram Membrane Tregs:Teffs 2:1; Fig.4B triangles, n=6) much more efficiently, than those cultured in standard conditions (monocultures, Fig.4A histogram Solo Tregs:Teffs 2:1; Fig.4B squares, n=6) and Tregs from cultures supplemented with ASC derived SNs (Fig.4A histogram SN Tregs:Teffs 2:1; Fig.4B circles, n=6). The difference in Teff suppression by Tregs from cocultures and those from standard monocultures was statistically significant for all tested Treg:Teff ratios (Mann-Whitney U test, 2:1 p=8x10$^{-3}$; 1:1 and 1:2 p=0.02; Fig.4). PI of conventional CD4$^+$ T cells (Teffs) in presence of the conditioned Tregs was 11%, 14.54% and 23.12% (median) lower for 2:1, 1:1 and 1:2 Tregs:Teffs ratios, respectively as compared with the values observed for Tregs expanded at standard culture conditions (monocultures). In addition, for the following Treg:Teff ratios 2:1, 1:1 and 1:2 PI of Teffs exposed to Tregs conditioned in the cocultures was at least 38%, 33% and 29% lower, respectively than PI of Teffs stimulated alone (positive control). Suppression of Teff proliferation by Tregs is crucial for their therapeutic effect. By the limitation of immune cell proliferation Tregs are able to restrain abnormal and/or detrimental immune responses, like autoimmunity, chronic inflammation, GVHD, allergic reactions or graft rejection.

Evaluation of eATP, eAMP and eADO degradation

After 7-day coculture, samples of Tregs expanded in presence of membrane separated ASCs, those from standard culture conditions (monoculture) and Tregs cultured in medium supplemented with ASC derived SNs were collected for assessment of their potential for degradation of eATP, eAMP, and eADO. The cells were distributed into 5 ml polystyrene tubes (1x10$^6$ Tregs/tube), washed twice with Hank's balanced salt solution (HBSS) and preincubated in 1 ml of HBSS (pH 7.4) with HEPES (1.25 ml of HEPES/50 ml of HBSS) and glucose (0.05 g of glucose/50 ml of HBSS). At this step also EHNA (1$\mu$l/1000$\mu$l HBSS) was added into the tubes where degradation of eATP and eAMP was measured to block adenosine deaminase activity and cells were incubated for 15 min at 37°C with gentle agitation. Subsequently, eATP, eAMP, or eADO was added at a final concentration of 50 $\mu$M and 70 $\mu$l of supernatants were collected at 0, 5, 15 and 30 minute time points. During the incubation period the cells were kept at 37°C and gently agitated. Each time the collection of SNs was preceded by centrifugation step (2 min., 1500 RPM) to eliminate the risk of cell collection that could affect the final results. The SNs were frozen immediately and stored at -80°C until analysis of the concentration of nucleotides and their catabolites with high-performance liquid chromatography (HPLC). The concentration of nucleotides and their catabolites was analysed with reverse-phase HPLC. The chromatographic system consisted of a Thermo Finnigan Surveyor autosampler and MS pump with UV6000RP Detector (Thermo Finnigan, UK). The analytical column (150×2.1 mm) Kinetex C18 with a 2.6-$\mu$m particle size (Phenomenex, USA) was used. The following chromatographic conditions were applied: buffer A was 122 mM potassium dihydrogen phosphate, 26.4 mM dipotassium hydrogen phosphate, and 150 mM potassium chloride; buffer B was a 15% (v/v) acetonitrile in buffer A. The amount of buffer B changed from 0% to 1% in 0.1 min, 3% in 3.3 min, 35% in 7.3 min, 100% in 9 min to 11.5 min, and 0% B in 11.6 min. The reequilibration time was 3.4 min, resulting in a cycle time of 15 min between injections. The flow rate was 200 $\mu$l/min, and the volume of injection was 2 $\mu$l. The analytical column was maintained at 23°C. Peaks were monitored by absorption at 254 nm. Data were collected and processed by Thermo Scientific Xcalibur Software (v.2.0, ThermoScientific, USA). The activity of eATP, eAMP, and eADO degrading enzymes was measured in nmol/min/1x10$^6$ cells.

The rates of eATP, eAMP and eADO degradations are depicted as INDEXes (Fig.5). The values reached by Tregs derived from monocultures were treated as a standard and thus set to 100% for each experiment. Consequently, the rates of eATP, eAMP and eADO hydrolysis by Tregs cultured in presence of membrane separated ASCs and those expanded in medium supplemented with SNs derived from ASC monocultures were converted adequately for each experiment and are shown in % in relation to the standard.

Tregs expanded in presence of membrane separated ASCs showed accelerated degradation of eATP (Fig.5A; MEMBRANE; n=4) and eAMP (Fig.5B; MEMBRANE; n=4) that are known mediators of inflammation as compared with Tregs expanded alone in basic culture medium (SOLO; Mann-Whitney U test, n=4, p=0.02 for degradation of both eATP and eAMP). We found that 1 x10$^6$ of conditioned Tregs degraded 26% (median) more nmol of eATP and 244.4% (median) more nmol of eAMP during 1 min., than Tregs expanded in standard culture conditions (monoculture). When the lowest values were compared we observed that 1 x10$^6$ of the conditioned Tregs degraded at least 3.9% more nmol of eATP and at least 163.5% more nmol of eAMP during 1 min., than Tregs expanded in standard culture conditions (monoculture). In addition, Tregs expanded in presence of membrane separated ASCs showed 212% (median) more efficient degradation of eAMP than Tregs from monocultures supplemented with ASC derived SNs (Fig.5B, SN; Mann-Whitney U test, n=4, p=0.02). Simultaneously, Tregs expanded in presence of membrane separated ASCs did not show accelerated degradation of eADO as compared with the two other tested conditions (Fig.5C). Therefore, contact with membrane separated ASCs increased significantly Treg capacity for generation and accumulation of eADO. This biological effect is of great importance for Treg immunosuppressive potential and efficacy of Treg based therapy. In addition, enhanced degradation of eAMP into adenosine was associated with stronger suppression of Teff proliferation that was manifested as a lower proliferation index of Teffs (Spearman's rank correlation, R= - 0.48, p=3x10$^{-3}$; correlation was calculated for all studied culture conditions and all tested Treg:Teff ratios together, n=36). No correlation was found between eADO degradation and suppression of Teff proliferation by Tregs.

Analysis of cytokine secretion

Each 24h and 48h after plating of the cells, SNs were collected from the following culture conditions: 1). Treg monocultures (n=6), 2). ASCs monocultures (n=6), and 3). Treg-ASC cocultures (n=6), where both cell types were separated with membranes (0.4 $\mu$m pore size) enabling bidirectional diffusion of mediators but not direct cell-to-cell contact. Immediately after collection, SNs were frozen and stored at -80°C until analysis. Concentrations of 48 mediators: IL-21, HGF (hepatocyte growth factor), IL-35, sCD40L, EGF (epidermal growth factor), eotaxin, FGF-2 (fibroblast growth factor), FLT-3L (FMS-like tyrosine kinase 3 ligand), fractalkine, G-CSF (granulocyte stimulating factor), GM-CSF (granulocyte-macrophage colony stimulating factor), GRO$\alpha$ (growth-related oncogene $\alpha$), IFN$\alpha$2 (interferon $\alpha$2), IFN$\gamma$ (interferon $\gamma$), IL-1$\alpha$, IL-1$\beta$, IL-1RA (IL-1 receptor antagonist), IL-2, IL-3, IL-4, IL-5, IL-6, IL-7, IL-8, IL-9, IL-10, IL-12p40, IL-12p70, IL-13, IL-15, IL-17A, IL-17E/IL-25, IL-17F, IL-18, IL-22, IL-27, IP-10 (interferon $\gamma$-induced protein 10), MCP-1/CCL2 (monocyte chemoattractant protein-1), MCP-3, M-CSF (macrophage colony-stimulating factor), MDC/CCL22 (macrophage-derived chemokine), MIG/CXCL9 (monokine induced by IFN$\gamma$), MIP-1$\alpha$/CCL3 (macrophage inflammatory protein-la), MIP-1$\beta$, PDGF-AA (platelet-derived growth factor AA), PDGF-AB/BB, RANTES/CCL5 (regulated upon activation, normal T cell expressed and secreted), TGF$\alpha$ (transforming growth factor $\alpha$), TNF$\alpha$ (tumour necrosis factor $\alpha$), TNF$\beta$, VEGF-A (vascular endothelial growth factor A) were measured with Human Cytokine/Chemokine/Growth Factor Panel A 48 Plex Kit (Merck) and analyzed with luminex (MAGPIX, Merck Millipore) according to the manufacturer instructions. In addition levels of HGF (hepatic growth factor, BIOLIM), IL21 (BioLegend), and IL35 (BIOLIM) were measured with ELISA..

It was found that cytokine concentrations measured after 24h and 48h in supernatants from Treg (n=6) and ASC (n=6) monocultures differed significantly from each other (Table 1; Tregs Solo 24h, Tregs Solo 48h, ASCs Solo 24h, and ASCs Solo 24h, respectively) and from SNs derived from Tregs cultured in presence of membrane separated ASCs (n=6; Table 1; Membrane 24h and Membrane 48h). Statistical differences between the groups were calculated with Mann-Whitney U test and $p < 0.05$ for comparisons between Membrane 24h vs Tregs Solo 24h v and Membrane 48h vs Tregs Solo 48h are marked with "*" in Table 1. At the same time $p < 0.05$ for comparisons between Membrane 24h vs ASCs Solo 24h and Membrane 48h vs ASCs Solo 48h are marked with "§". Analogically statistically significant ($p < 0.05$) differences between Tregs Solo 24h vs ASCs Solo 24h and Tregs Solo 48h vs ASCs Solo 48h are marked with "**".

These data show that each of the tested culture conditions present an unique microenvironment that affects Treg phenotype and function in a different manner. Notably, cytokine levels in SNs derived from Tregs cultured in presence of membrane separated ASCs were not a direct sum of these cytokine concentrations in SNs derived from Treg and ASC monocultures, but deviated significantly from both. These data indicate that the microenvironment in the Treg-ASC cocultures was shaped by bidirectional interplay between these two cell types. At this point it has to be also mentioned that the results for all experiments were consistent, despite ASCs derived from allogeneic donors and each time Tregs and ASCs from different unrelated donors were cocultured. These observations justify using of allogeneic ASCs for enhancement of Treg therapeutic potential.

The present invention relates to the conditioned regulatory T cell population (Tregs) with following characterization and therapeutic potential, listed below:
Proliferate intensively and preserve their phenotype, including expression of CD4, FoxP3, CD25, CTLA-4, Helios and CD39.

As compared with the cells expanded in standard culture conditions (monoculture) are characterized by significantly higher percentage of CD25$^{High}$ cells (Fig. 2); high expression of CD25 (IL2R$\alpha$) makes Tregs more competitive for IL-2 binding and thus inhibits proliferation of conventional T cells (Teffs) by IL-2 deprivation. The population is characterized by higher expression of CD73 ectonucleotidase, than the cells expanded in standard culture conditions (monoculture; Fig.3).

Present significantly stronger potential for restoring immune tolerance and suppression of deviant immune responses, than Tregs cultured in standard culture conditions (monocultures) and those from monocultures supplemented with ASC derived SNs.

The population is characterized by significantly enhanced degradation of proinflammatory eATP and eAMP resulting in accumulation of eADO- known to prevent autoimmunity and restore immune tolerance (Fig.5). The cells show significantly stronger inhibition of proliferation of conventional T cells (Fig.4) that is crucial for limitation of deviant immune responses (e.g. autoimmunity, graft rejection, allergic reactions, exaggerated inflammatory responses and GVHD).

The Tregs can be used for clinical therapies of adverse immune reactions that are defined as: autoimmune diseases, transplant rejection, allergic reactions, exaggerated inflammatory responses and graft-versus-host disease.

The Tregs are polyclonal or antigen-specific cells.

Features listed above are of great importance for efficacy of Treg based therapies. Only administration of fully functional Tregs can provide beneficial effect of Treg based therapy.

The present invention relates also to the method of conditioning and expansion of conditioned regulatory T cell population (Tregs) and is characterized by the following:
It enables to obtain ≈20% more therapeutically active Tregs during defined period of time, than standard culture methods (Fig.1).

It enables to obtain Tregs with significantly stronger potential for restoring immune tolerance and suppression of deviant immune responses, than Tregs cultured in standard culture conditions (monocultures) and those from monocultures supplemented with ASC derived SNs.

The Tregs obtained with this method can be used for clinical therapies of adverse immune reactions that are defined as: autoimmune diseases, transplant rejection, allergic reactions, exaggerated inflammatory responses and graft-versus-host disease.

The method is not associated with risk of adverse effects that can potentially occur when Treg cultures are supplemented with chemical compounds/drugs or other cells in direct cell-to-cell contact in aim to improve the cell stability, function or proliferation. In the current method membrane with 0.4 μm pore size prevents unintentional transfer of allogeneic ASCs into Treg recipient. In the presented example the membrane was made from polyethylene terephthalate (PET), but the type of material the membrane was made of is not the limitation of the invention.

The method enables reduction of the time for cell production for about 20%, or to obtain ≈ 20% higher number of Tregs during 7- day culture. Thus, Treg based therapy can be provided to higher number of patients or higher therapeutic doses of Tregs can be obtained for the treatment.

As compared to the previously described techniques for potentiation of Treg expansion/function the current method is cost effective as ASCs are obtained from adipose tissue of healthy unrelated donors. The adipose tissue is collected during liposuction procedure and thus is recognised as biological waste.

In the case of clinical application the method enables to obtain better therapeutic effect due to production of Tregs with significantly higher immunosuppressive and tolerance inducing potential, than conventional methods. In addition, the final product does not contain traces of cytotoxic and potentially dangerous drugs used for Treg expansion in previously described protocols (e.g. azacitidine). Moreover due to separation of ASCs and Tregs with a membrane, no risk of transfer of allogeneic ASCs to the Treg recipient exists.

Table 1

| Cytokine | Tregs Solo 24h | Tregs Solo 48h | Membrane 24h | Membrane 48h | ASCs Solo 24h | ASCs Solo 48h |
|---|---|---|---|---|---|---|
| IL-21 | 0.00 | 0.00 | 0.00 | 0.00 (0.00-233.16 ) | 0.00 | 0.00 |
| HGF | 0.00 | 0.00 (0.00-167.16) | 0.00 | 0.00 | 0.00 | 0.00 |
| IL-35 | 11.97 (0.00-28.21)** | 32.79 (0.00-39.05) ** | ≥1049.0* | ≥1049.0* | 1049.00 (838.56-1049.00) | ≥1049.00 |
| sCD40L | 0.00 | 0.00 (0.00-6.58) | 5.43 (0.00-8.81) | 7.27 (0.00-8.59) | 0.00 | 0.00 |
| EGF | 0.89 (0.71-1.15) | 1.10 (0.95-1.37) | 1.48 (1.28-1.96)*§ | 1.87 (1.60-2.29)* § | 0.95 (0.83-1.23) | 0.98 (0.83-1.12) |
| Eotaxin | 1.46 (1.23-1.84) | 1.98 (1.73-2.47) | 2.27 (1.78-3.87)* § | 2.68 (2.27-8.22)* § | 1.58 (1.30-1.84) | 1.68 (1.43-2.06) |
| FGF-2 | 0.61 (0.56-0.71) ** | 0.74 (0.61-0.78) | 1.19 (0.80-1.49)* | 1.15 (0.84-1.46)* § | 1.19 (0.67-3.79) | 0.72 (0.64-1.13) |
| FLT-3L | 3.24 (2.82-3.55) ** | 6.00 (0.55-6.81) ** | 4.32 (3.12-5.23)* § | 8.28 (6.26-11.10)* § | 0.56 (0.48-0.62) | 0.55 (0.53-0.58) |
| Fractalkine | 0.59 (0.00-0.97) | 0.72 (0.65-0.97) | 1.06 (0.84-1.26)* § | 1.22 (0.97-1.74)* § | 0.69 (0.00-0.84) | 0.75 (0.65-1.00) |
| G-CSF | 0.50 | 0.67 (0.39- | 18.89 | 274.07 | 0.76 | 0.98 |

| | | | | | | |
|---|---|---|---|---|---|---|
| | (0.34-0.73) | 0.98) | (1.38-95.37)* § | (2.54-512.29)* § | (0.50-0.85) | (0.55-2.69) |
| GM-CSF | 18.28 (15.47-34.26) ** | 26.40 (1.21-56.60) ** | 22.93 (11.15-30.85 ) § | 41.43 (20.97-64.46) § | 0.00 (0.00-0.99) | 0.43 (0.00-1.21) |
| GROα | 0.35 (0.25-1.66) ** | 0.43 (0.35-18.97) ** | 32.15 (13.01-390.98)* § | 312.40 (38.20-1015.00)* § | 7.44 (2.82-27.96) | 18.17 (4.16-74.52) |
| IFNα2 | 1.37 (1.03-1.69) | 1.45 (1.01-1.91) | 1.65 (1.60-2.29) *§ | 1.91 (1.73-2.64)* § | 1.15 (0.81-1.43) | 1.23 (1.01-1.32) |
| IFNγ | 61.02 (14.76-134.43) ** | 91.45 (1.78-217.73) ** | 69.38 (15.40-81.99) § | 120.50 (13.82-215.92) § | 1.34 (1.150-1.74) | 1.40 (1.240-1.78) |
| IL-1α | 0.15 (0.00-1.08) | 0.33 (0.00-1.38) | 0.36 (0.00-0.51) | 0.38 (0.00-0.69) § | 0.00 | 0.00 |
| IL-1β | 1.71 (1.61-2.40) | 2.28 (1.80-2.97) ** | 2.92 (2.00-3.85)* § | 3.18 (2.51-4.06)* § | 1.59 (1.420-2.10) | 1.74 (1.470-2.25) |
| IL-1RA | 1.70 (1.60-2.16) | 1.95 (1.63-2.84) | 2.28 (1.76-2.84)* § | 3.55 (2.18-5.03)* § | 1.66 (1.560-1.78) | 1.80 (1.700-1.96) |
| IL-2 | 11719.5 (10848.0-13148.0) | 11615.5 (11112.0-12728.0) | 12357.50 (11580.0-13170.0) | 11536.0 (11411.0-12630.0) | 12059.00 (9157.0-12795.0) | 12101.50 (9280.0-12638.0) |
| IL-3 | 8.37 (3.14-19.80) ** | 11.61 (0.00-26.11) ** | 8.45 (5.28-19.32) § | 17.07 (7.71-1.27) § | 0.00 (0.00-1.18) | 0.00 (0.00-0.89) |
| IL-4 | 23.82 (21.46-233.71) ** | 17.42 (8.29-147.57) | 25.97 (19.47-156.36)§ | 14.74 (13.53-114.94) § | 9.04 (7.94-9.63) | 8.69 (8.29-9.47) |
| IL-5 | 263.05 (138.37-4492.00) ** | 479.98 (0.38-5173.00) ** | 441.27 (177.61-3178.00) § | 565.33 (288.07-4066.00) § | 0.43 (0.38-0.48) | 0.43 (0.38-0.48) |
| IL-6 | 4.55 (0.85-12.18) ** | 14.31 (4.30-4506.00) | 3729.00 (1595.0-9405.0)* § | 8779.50 (4605.0-10574.0)* § | 1342.00 (1234.00-3718.00) | 1682.00 (1465.00-4506.00) |
| IL-7 | 0.50 (0.42-0.72) ** | 0.59 (0.46-0.84) | 0.77 (0.51-1.62)* § | 1.24 (1.00-2.31)* § | 0.32 (0.25-0.57) | 0.39 (0.28-0.84) |

| | | | | | | |
|---|---|---|---|---|---|---|
| IL-8 | 16.83 (11.74-42.19) ** | 29.12 (15.50-130.52) ** | 2250.50 (841.84-5648.00)* § | 4927.00 (1555.00-6890.00) * § | 228.35 (67.31-405.28) | 337.58 (80.89-591.61) |
| IL-9 | 51.89 (44.79-174.95) ** | 56.28 (37.89-189.85) ** | 60.38 (51.66-129.15) § | 71.23 (50.57-150.12) § | 37.72 (36.32-38.52) | 37.93 (35.51-38.39) |
| IL-10 | 331.08 (60.55-1460.0) ** | 333.67 (0.52-1633.0) ** | 390.79 (121.6-995.76) § | 328.10 (117.12-1231.0) | 0.36 (0.210-0.45) | 0.39 (0.31-0.52) |
| IL-12p40 | 0.74 (0.61-0.88) ** | 0.99 (0.74-1.46) | 2.15 (1.38-2.54)* § | 2.70 (2.54-4.30)* § | 1.06 (0.810-1.31) | 1.09 (0.95-1.46) |
| IL-12p70 | 0.59 (0.50-0.84) | 0.58 (0.46-0.73) | 0.74 (0.57-1.07) § | 1.00 (0.69-1.38)* § | 0.50 (0.420-0.57) | 0.48 (0.42-0.53) |
| IL-13 | 20.51 (16.91-147.07) ** | 29.75 (0.68-178.86) ** | 19.40 (16.58-95.20) § | 22.17 (16.86-120.01) § | 0.64 (0.600-0.78) | 0.68 (0.60-0.80) |
| IL-15 | 0.48 (0.41-0.57) ** | 0.57 (0.46-0.65) ** | 0.67 (0.57-1.76)* § | 1.30 (0.77-2.30)* § | 0.41 (0.36-0.47) | 0.45 (0.410-0.49) |
| IL-17A | 8.77 (1.91-60.33) ** | 7.66 (1.58-134.62) ** | 24.24 (4.27-86.20) § | 74.61 (4.39-156.33) § | 1.15 (0.880-1.58) | 1.32 (0.62-1.58) |
| IL-17E/ IL-25 | 0.17 (0.00-0.37) | 0.37 (0.00-0.57) ** | 0.50 (0.40-0.99)* § | 0.96 (0.42-1.80)* § | 0.00 | 0.00 |
| IL-17F | 0.00 | 0.00 | 0.00 | 0.00 | 0.00 | 0.00 |
| IL-18 | 0.49 (0.44-0.64) | 0.67 (0.54-0.74) ** | 0.80 (0.47-0.93)* § | 1.20 (0.86-1.25)* § | 0.22 (0.00-0.54) | 0.47 (0.44-0.54) |
| IL-22 | 0.76 (0.66-0.93) | 0.80 (0.77-1.17) | 1.43 (1.03-2.26)* § | 2.09 (1.36-2.66)* § | 0.89 (0.71-1.14) | 1.04 (0.68-1.17) |
| IL-27 | 4.68 (4.47-4.88) | 4.78 (4.32-5.27) | 5.54 (5.39-6.15)* § | 6.70 (5.63-7.49)* § | 4.78 (4.47-4.88) | 4.61 (4.10-5.01) |
| IP-10 | 0.94 (0.63-1.35) ** | 1.14 (0.68-2.27) ** | 60.78 (28.01-920.58)* § | 378.58 (79.18-1600.00) * § | 0.55 (0.45-0.86) | 0.57 (0.49-0.81) |
| MCP-1 | 0.87 (0.76-1.15) ** | 1.31 (0.89-186.11) | 677.03 (304.0-1478.00)* § | 1962.00 (1639.00-2127.00) * § | 33.69 (12.76-102.00) | 91.29 (44.72-186.11) |

| | | | | | | |
|---|---|---|---|---|---|---|
| MCP-3 | 1.65 (1.39-1.82) | 1.87 (1.71-5.74) | 13.65 (4.68-54.79)* § | 84.45 (13.12-138.26)* § | 1.76 (1.45-4.82) | 2.59 (1.66-5.74) |
| M-CSF | 3.35 (2.68-10.13) ** | 7.61 (2.83-19.87) ** | 8.43 (6.98-13.60) § | 18.50 (13.31-22.71) § | 1.35 (0.780-1.90) | 2.56 (1.41-3.18) |
| MDC/CCL22 | 3.08 (1.64-6.53) ** | 9.36 (0.83-23.45) ** | 1.61 (1.30-3.68) § | 3.72 (1.68-13.73) § | 0.70 (0.00-1.04) | 0.80 (0.00-1.20) |
| MIG | 8.75 (4.33-23.11) ** | 22.86 (1.68-53.98) ** | 18.78 (11.94-353.56) § | 92.32 (34.77-1583.0)* § | 1.41 (1.28-1.74) | 1.47 (1.41-1.68) |
| MIP-1α | 53.47 (18.36-148.81) ** | 168.19 (5.33-565.77) ** | 54.96 (32.85-110.54) § | 197.03 (111.89-411.17) § | 2.22 (1.97-3.76) | 2.40 (2.24-5.33) |
| MIP-1β | 116.10 (58.01-427.83) ** | 372.28 (14.63-2069.0) ** | 123.54 (89.97-287.54) § | 463.10 (179.66-1260.0) § | 3.54 (3.32-14.38) | 3.77 (3.10-14.63) |
| PDGF-AA | 1.58 (0.85-1.85) | 3.13 (1.39-4.25) | 2.26 (1.79-5.47)* | 3.41 (1.65-8.49) | 1.65 (1.120-6.74) | 1.37 (1.20-6.11) |
| PDGF-AB/BB | 1.55 (1.37-2.69) | 2.21 (1.61-2.63) | 2.90 (1.97-6.40)* | 3.45 (2.33-4.96)* § | 1.97 (1.55-3.05) | 2.03 (1.61-2.81) |
| RANTES | 103.83 (69.40-246.02) ** | 564.80 (13.13-1446.00) ** | 110.98 (61.77-154.20) § | 798.21 (189.85-1103.00) § | 1.28 (0.40-5.73) | 3.02 (0.63-13.13) |
| TGFα | 0.00 (0.00-0.60) ** | 0.56 (0.42-0.66) ** | 1.36 (1.15-3.39)* | 2.53 (1.36-3.63)* § | 0.99 (0.54-2.22) | 1.08 (0.58-1.48) |
| TNFα | 20.78 (7.29-26.63) ** | 27.16 (3.08-39.70) ** | 28.27 (13.00-44.61) § | 32.52 (12.04-53.09) § | 2.10 (1.92-3.30) | 2.21 (2.07-3.08) |
| TNFβ | 17.18 (5.86-43.42) ** | 9.79 (2.56-51.27) | 24.57 (11.20-63.71) § | 21.45 (10.88-76.26) § | 5.06 (4.37-8.32) | 6.18 (5.00-8.05) |
| VEGF-A | 0.95 (0.79-2.79) ** | 1.14 (0.84-41.35) ** | 7.00 (2.93-24.58)* § | 26.00 (10.48-53.11)* | 25.63 (14.83-36.78) | 32.98 (21.05-62.53) |

References - state of the art cited above.

1. D. A. Vignali, L. W. Collison, C. J. Workman, How regulatory T cells work. Nat Rev Immunol 8, 523-532 (2008).

2. E. Gambineri, T. R. Torgerson, H. D. Ochs, Immune dysregulation, polyendocrinopathy, enteropathy, and X-linked inheritance (IPEX), a syndrome of systemic autoimmunity caused by mutations of FOXP3, a critical regulator of T-cell homeostasis. Curr Opin Rheumatol 15, 430-435 (2003).

3. N. Pilat et al., Treg-mediated prolonged survival of skin allografts without immunosuppression. Proc Natl Acad Sci U S A 116, 13508-13516 (2019).

4. R. D. Danby et al., High proportions of regulatory T cells in PBSC grafts predict improved survival after allogeneic haematopoietic SCT. Bone Marrow Transplant 51, 110-118 (2016).

5. M. Sharma et al., Regulatory T Cells License Macrophage Pro-Resolving Functions During Atherosclerosis Regression. Circ Res 127, 335-353 (2020).

6. N. Marek-Trzonkowska et al., Therapy of type 1 diabetes with CD4(+)CD25(high) CD127-regulatory T cells prolongs survival of pancreatic islets - Results of one year follow-up. Clinical Immunology 153, 23-30 (2014).

7. R. W. M. Kempkes, I. Joosten, H. Koenen, X. He, Metabolic Pathways Involved in Regulatory T Cell Functionality. Front Immunol 10, 2839 (2019).

8. S. Létourneau, C. Krieg, G. Pantaleo, O. Boyman, IL-2- and CD25-dependent immunoregulatory mechanisms in the homeostasis of T-cell subsets. J Allergy Clin Immunol 123, 758-762 (2009).

9. T. Chinen et al., An essential role for the IL-2 receptor in T(reg) cell function. Nat Immunol 17, 1322-1333 (2016).

10. M. Vuerich, R. P. Harshe, S. C. Robson, M. S. Longhi, Dysregulation of Adenosinergic Signaling in Systemic and Organ-Specific Autoimmunity. Int J Mol Sci 20, (2019).

11. E. Castillo-Leon, S. Dellepiane, P. Fiorina, ATP and T-cell-mediated rejection. Curr Opin Organ Transplant 23, 34-43 (2018).

12. F. Morandi, A. L. Horenstein, R. Rizzo, F. Malavasi, The Role of Extracellular Adenosine Generation in the Development of Autoimmune Diseases. Mediators Inflamm 2018, 7019398 (2018).

13. M. Durand et al., Increased degradation of ATP is driven by memory regulatory T cells in kidney transplantation tolerance. Kidney Int 93, 1154-1164 (2018).

14. L. Antonioli, C. Blandizzi, P. Pacher, G. Haskó, Immunity, inflammation and cancer: a leading role for adenosine. Nat Rev Cancer 13, 842-857 (2013).

15. L. Airas, J. Niemelä, G. Yegutkin, S. Jalkanen, Mechanism of action of IFN-beta in the treatment of multiple sclerosis: a special reference to CD73 and adenosine. Ann N Y Acad Sci 1110, 641-648 (2007).

16. J. Niemelä et al., IFN-beta regulates CD73 and adenosine expression at the blood-brain barrier. Eur J Immunol 38, 2718-2726 (2008).

17. R. S. Peres et al., Low expression of CD39 on regulatory T cells as a biomarker for resistance to methotrexate therapy in rheumatoid arthritis. Proc Natl Acad Sci U S A 112, 2509-2514 (2015).

18. S. Botta Gordon-Smith, S. Ursu, S. Eaton, H. Moncrieffe, L. R. Wedderburn, Correlation of low CD73 expression on synovial lymphocytes with reduced adenosine generation and higher disease severity in juvenile idiopathic arthritis. Arthritis Rheumatol 67, 545-554 (2015).

19. D. J. Lee, A. W. Taylor, Recovery from experimental autoimmune uveitis promotes induction of antiuveitic inducible Tregs. J Leukoc Biol 97, 1101-1109 (2015).

20. L. Antonioli, C. Blandizzi, B. Csóka, P. Pacher, G. Haskó, Adenosine signalling in diabetes mellitus--pathophysiology and therapeutic considerations. Nat Rev Endocrinol 11, 228-241 (2015).

21. C. Blume et al., Autoimmunity in CD73/Ecto-5'-nucleotidase deficient mice induces renal injury. PLoS One 7, e37100 (2012).

22. H. Tsukamoto et al., Deficiency of CD73/ecto-5'-nucleotidase in mice enhances acute graft-versus-host disease. Blood 119, 4554-4564 (2012).

23. G. D. Whitehill et al., Adenosine Selectively Depletes Alloreactive T Cells to Prevent GVHD While Conserving Immunity to Viruses and Leukemia. Mol Ther 24, 1655-1664 (2016).

24. P. Trzonkowski et al., First-in-man clinical results of the treatment of patients with graft versus host disease with human ex vivo expanded CD4+CD25+CD127- T regulatory cells. Clin Immunol 133, 22-26 (2009).

25. N. Marek-Trzonkowska et al., Administration of CD4(+)CD25(high)CD127(-) Regulatory T Cells Preserves beta-Cell Function in Type 1 Diabetes in Children. Diabetes Care 35, 1817-1820 (2012).

26. P. Trzonkowski et al., Hurdles in therapy with regulatory T cells. Sci Transl Med 7, 304ps318 (2015).

27. M. Di Ianni et al., Tregs prevent GVHD and promote immune reconstitution in HLA-haploidentical transplantation. Blood 117, 3921-3928 (2011).

28. P. Hoffmann et al., Loss of FOXP3 expression in natural human CD4+CD25+ regulatory T cells upon repetitive in vitro stimulation. Eur J Immunol 39, 1088-1097 (2009).

29. A. Visperas, D. A. Vignali, Are Regulatory T Cells Defective in Type 1 Diabetes and Can We Fix Them? J Immunol 197, 3762-3770 (2016).

30. J. A. Bluestone et al., Type 1 diabetes immunotherapy using polyclonal regulatory T cells. Sci Transl Med 7, 315ra189 (2015).

31. N. Marek-Trzonkowska et al., Mild hypothermia provides Treg stability. Sci Rep 7, 11915 (2017).

32. N. Safinia et al., Cell Therapy in Organ Transplantation: Our Experience on the Clinical Translation of Regulatory T Cells. Front Immunol 9, 354 (2018).

33. A. L. Putnam et al., Expansion of human regulatory T-cells from patients with type 1 diabetes. Diabetes 58, 652-662 (2009).

34. K. L. Hippen et al., Massive ex vivo expansion of human natural regulatory T cells (T(regs)) with minimal loss of in vivo functional activity. Sci Transl Med 3, 83ra41 (2011).

35. S. Landman et al., DNA Methyltransferase Inhibition Promotes Th1 Polarization in Human CD4(+)CD25(high) FOXP3(+) Regulatory T Cells but Does Not Affect Their Suppressive Capacity. J Immunol Res 2018, 4973964 (2018).

36. M. Ryba et al., Anti-TNF rescue CD4(+)Foxp3(+) regulatory T cells in patients with type 1 diabetes from effects mediated by TNF. Cytokine 55, 353-361 (2011).

37. Z. M. Liu, K. P. Wang, J. Ma, S. Guo Zheng, The role of all-trans retinoic acid in the biology of Foxp3+ regulatory T cells. Cell Mol Immunol 12, 553-557 (2015).

38. M. Pikula, N. Marek-Trzonkowska, A. Wardowska, A. Renkielska, P. Trzonkowski, Adipose tissue-derived stem cells in clinical applications. Expert Opin Biol Ther 13, 1357-1370 (2013).

39. V. Sénécal et al., Production of IL-27 in multiple sclerosis lesions by astrocytes and myeloid cells: Modulation of local immune responses. Glia 64, 553-569 (2016).

40. X. T. Lima et al., Frequency and characteristics of circulating CD4(+) CD28(null) T cells in patients with psoriasis. Br J Dermatol 173, 998-1005 (2015).

41. Q. Mu, H. Zhang, X. M. Luo, SLE: Another Autoimmune Disorder Influenced by Microbes and Diet? Front Immunol 6, 608 (2015).

42. W. Orent et al., Rheumatoid arthritis-associated RBPJ polymorphism alters memory CD4+ T cells. Hum Mol Genet 25, 404-417 (2016).

43. S. Gupta, Immunotherapies in diabetes mellitus type 1. Med Clin North Am 96, 621-634, xi (2012).

44. I. Rama, J. M. Grinyó, Malignancy after renal transplantation: the role of immunosuppression. Nat Rev Nephrol 6, 511-519 (2010).

45. Á. Prókai et al., Calcineurin-inhibition Results in Upregulation of Local Renin and Subsequent Vascular Endothelial Growth Factor Production in Renal Collecting Ducts. Transplantation 100, 325-333 (2016).

46. T. Berney, A. Secchi, Rapamycin in islet transplantation: friend or foe? Transpl Int 22, 153-161 (2009).

47. K. Zhao et al., Dynamic regulation of effector IFN-γ-producing and IL-17-producing T cell subsets in the development of acute graft-versus-host disease. Mol Med Rep 13, 1395-1403 (2016).

48. R. A. Panettieri, Jr., R. Covar, E. Grant, E. V. Hillyer, L. Bacharier, Natural history of asthma: persistence versus progression-does the beginning predict the end? J Allergy Clin Immunol 121, 607-613 (2008).

49. Q. Tang, J. A. Bluestone, Regulatory T-cell therapy in transplantation: moving to the clinic. Cold Spring Harb Perspect Med 3, (2013).

## Claims

1. Population of CD4$^+$FoxP3$^+$ regulatory T cells (Tregs) **characterized by** high expression of CD25 and enhanced immunosuppressive function while the population is obtained as a result of coculture in indirect contact with adipose tissue derived mesenchymal stem cells (ASCs) from allogeneic donors wherein Tregs and ASCs are cocultured in indirect contact in two compartments separated from each other with a membrane with pore size ≤0.4μm impermeable for cells and extracellular vesicles with the diameter >0.4 μm but permeable for secreted soluble molecules that pass freely through the membrane between the two culture compartments.

2. Population according to claim 1, wherein Tregs are obtained after permanent or temporary coculture in indirect contact with allogeneic human ASCs that lasts at least 3 days

3. Population according to claims 1-2, wherein at least 85% of the Tregs is positive for CD25.

4. Population according to claims 1-3, wherein the Tregs are polyclonal or monoclonal population.

5. Population according to claims 1-4 wherein the Tregs decrease proliferation index (PI) of conventional CD4$^+$ T cells (Teffs) at least 38% or 33% or 29% when Tregs and Teffs are present at the following Treg:Teff ratios 2:1, 1:1 and 1:2 respectively, as compared to Teffs stimulated alone.

6. Population according to claim 1-5, wherein 1 x10$^6$ of the conditioned Tregs degrades at least 3.9% more nmol of extracellular adenosine triphosphate (eATP) and 163.5% more nmol of extracellular adenosine monophosphate (eAMP) during 1 min., than Tregs expanded in standard culture conditions (monoculture).

7. Population disclosed in claim 1-6 for use in the treatment of autoimmune diseases, and/or for amelioration or suppression of adverse immune responses, preferably graft rejection, allergic reactions, graft versus host disease (GVHD), exaggerated inflammatory responses.

8. Method for in vitro expansion of population of CD4$^+$FoxP3$^+$ regulatory T cells (Tregs) **characterized by** high expression of CD25 and enhanced immunosuppressive function while the population is obtained as a result of coculture in indirect contact with adipose tissue derived mesenchymal stem cells (ASCs) from allogeneic donors wherein Tregs and ASCs are cocultured in indirect contact in two compartments separated from each other with a membrane with pore size ≤0.4μm impermeable for cells and extracellular vesicles with the diameter >0.4 μm but permeable for secreted soluble molecules that pass freely through the membrane between the two culture compartments.

9. Method according to claim 8, wherein Tregs are obtained after permanent or temporary coculture in indirect contact with allogeneic human ASCs that lasts at least 3 days.

10. Method according to claims 8-9, wherein Tregs are polyclonal or monoclonal population.

Figure 1

Figure 2

Figure 3

Figure 4

Figure 5

Europäisches
Patentamt

European
Patent Office

Office européen
des brevets

## EUROPEAN SEARCH REPORT

Application Number

EP 20 20 2376

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CROP MEINDERT J. ET AL: "Human Adipose Tissue-Derived Mesenchymal Stem Cells Induce Explosive T-Cell Proliferation", STEM CELLS AND DEVELOPMENT, vol. 19, no. 12, 1 December 2010 (2010-12-01), pages 1843-1853, XP055788570, US ISSN: 1547-3287, DOI: 10.1089/scd.2009.0368 Retrieved from the Internet: URL:https://www.liebertpub.com/doi/pdfplus /10.1089/scd.2009.0368> | 1-5,8-10 | INV. C12N5/0783 |
| Y | * page 1850, left-hand column - right-hand column; figure 5 * | 6 | |
| X | NEGI NEEMA ET AL: "Effects of mesenchymal stromal cells on regulatory T cells: Current understanding and clinical relevance : MSC effects on T-reg", STEM CELLS, vol. 38, no. 5, 3 February 2020 (2020-02-03), pages 596-605, XP055788578, ISSN: 1066-5099, DOI: 10.1002/stem.3151 Retrieved from the Internet: URL:https://api.wiley.com/onlinelibrary/td m/v1/articles/10.1002%2Fstem.3151> * the whole document * | 1-5,8-10 | |

TECHNICAL FIELDS SEARCHED (IPC)

C12N

-/--

The present search report has been drawn up for all claims

| Place of search | Date of completion of the search | Examiner |
|---|---|---|
| Munich | 15 April 2021 | Armandola, Elena |

EPO FORM 1503 03.82 (P04C01)

page 1 of 5

Europäisches
Patentamt

European
Patent Office

Office européen
des brevets

# EUROPEAN SEARCH REPORT

Application Number

EP 20 20 2376

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ENGELA A. U. ET AL: "Human adipose-tissue derived mesenchymal stem cells induce functional de-novo regulatory T cells with methylated FOXP3 gene DNA", CLINICAL AND EXPERIMENTAL IMMUNOLOGY, vol. 173, no. 2, 4 July 2013 (2013-07-04), pages 343-354, XP055788563, GB ISSN: 0009-9104, DOI: 10.1111/cei.12120 * page 345, right-hand column; figure 3 * | 1-4,8-10 | |
| X | KRONSTEINER BARBARA ET AL: "Human Mesenchymal Stem Cells from Adipose Tissue and Amnion Influence T-Cells Depending on Stimulation Method and Presence of Other Immune Cells", STEM CELLS AND DEVELOPMENT, vol. 20, no. 12, 1 December 2011 (2011-12-01), pages 2115-2126, XP055791702, US ISSN: 1547-3287, DOI: 10.1089/scd.2011.0031 Retrieved from the Internet: URL:https://www.liebertpub.com/doi/pdf/10.1089/scd.2011.0031> * the whole document * | 1-4,8-10 | TECHNICAL FIELDS SEARCHED (IPC) |
| | -/-- | | |

The present search report has been drawn up for all claims

| Place of search | Date of completion of the search | Examiner |
|---|---|---|
| Munich | 15 April 2021 | Armandola, Elena |

EPO FORM 1503 03.82 (P04C01)

page 2 of 5

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | USHA SHALINI P ET AL: "In vitroallogeneic immune cell response to mesenchymal stromal cells derived from human adipose in patients with rheumatoid arthritis", CELLULAR IMMUNOLOGY, ACADEMIC PRESS, SAN DIEGO, CA, US, vol. 314, 12 January 2017 (2017-01-12), pages 18-25, XP029953285, ISSN: 0008-8749, DOI: 10.1016/J.CELLIMM.2017.01.008 * the whole document * | 1-4,8-10 | |
| X | WO 2010/052313 A1 (CELLERIX S A [ES]; BUESCHER DIRK [ES] ET AL.) 14 May 2010 (2010-05-14) * page 3 - page 4; figures * * page 27 - page 29 * | 7 | |
| X | EUN-SOL LEE ET AL: "Adoptive Transfer of Treg Cells Combined with Mesenchymal Stem Cells Facilitates Repopulation of Endogenous Treg Cells in a Murine Acute GVHD Model", PLOS ONE, vol. 10, no. 9, 22 September 2015 (2015-09-22), page e0138846, XP055700317, DOI: 10.1371/journal.pone.0138846 * the whole document * | 7 | TECHNICAL FIELDS SEARCHED (IPC) |

-/--

The present search report has been drawn up for all claims

| Place of search | Date of completion of the search | Examiner |
|---|---|---|
| Munich | 15 April 2021 | Armandola, Elena |

EPO FORM 1503 03.82 (P04C01)

page 3 of 5

Europäisches
Patentamt

European
Patent Office

Office européen
des brevets

**EUROPEAN SEARCH REPORT**

Application Number

EP 20 20 2376

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KHOSRAVI MARYAM ET AL: "Induction of CD4+CD25+Foxp3+regulatory T cells by mesenchymal stem cells is associated with RUNX complex factors", IMMUNOLOGIC RESEARCH, HUMANA PRESS, INC, US, vol. 66, no. 1, 16 November 2017 (2017-11-16), pages 207-218, XP036423728, ISSN: 0257-277X, DOI: 10.1007/S12026-017-8973-4 [retrieved on 2017-11-16] * Materials and Methods; figures * | 1-4,8-10 | |
| Y | LI YANG ET AL: "Bone marrow mesenchymal stem cells suppressing activation of allogeneic cytokine-induced killer/natural killer cells either by direct or indirect interaction : MSC suppress the activation of CIK/NK cells", CELL BIOLOGY INTERNATIONAL., vol. 39, no. 4, 26 January 2015 (2015-01-26), pages 435-445, XP055791706, GB ISSN: 1065-6995, DOI: 10.1002/cbin.10404 * Materials and Methods * | 1-4,8-10 | TECHNICAL FIELDS SEARCHED (IPC) |

-/--

The present search report has been drawn up for all claims

| Place of search | Date of completion of the search | Examiner |
|---|---|---|
| Munich | 15 April 2021 | Armandola, Elena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 4 of 5

Europäisches
Patentamt

European
Patent Office

Office européen
des brevets

# EUROPEAN SEARCH REPORT

Application Number

EP 20 20 2376

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DEAGLIO SILVIA ET AL: "Adenosine generation catalyzed by CD39 and CD73 expressed on regulatory T cells mediates immune suppression", JOURNAL OF EXPERIMENTAL MEDICINE, ROCKEFELLER UNIVERSITY PRESS, US, vol. 204, no. 6, 1 June 2007 (2007-06-01), pages 1257-1265, XP002485581, ISSN: 0022-1007, DOI: 10.1084/JEM.20062512 * the whole document * | 6 | |
| A | RUEDA CESAR M. ET AL: "Regulatory T-Cell-Mediated Suppression of Conventional T-Cells and Dendritic Cells by Different cAMP Intracellular Pathways", FRONTIERS IN IMMUNOLOGY, vol. 7, 2 June 2016 (2016-06-02), XP055794751, DOI: 10.3389/fimmu.2016.00216 | 6 | |
| T | AGNESE FIORI ET AL: "Human Adipose Tissue-Derived Mesenchymal Stromal Cells Inhibit CD4+ T Cell Proliferation and Induce Regulatory T Cells as Well as CD127 Expression on CD4+CD25+ T Cells", CELLS, vol. 10, 1 January 2021 (2021-01-01), pages 58-76, XP055788584, Retrieved from the Internet: URL:https://www.mdpi.com/2073-4409/10/1/58 > | | TECHNICAL FIELDS SEARCHED (IPC) |

The present search report has been drawn up for all claims

| Place of search | Date of completion of the search | Examiner |
|---|---|---|
| Munich | 15 April 2021 | Armandola, Elena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 20 2376

This annex lists the patent family members relating to the patent documents cited in the above-mentioned European search report.
The members are as contained in the European Patent Office EDP file on
The European Patent Office is in no way liable for these particulars which are merely given for the purpose of information.

15-04-2021

| Patent document cited in search report | Publication date | Patent family member(s) | Publication date |
|---|---|---|---|
| WO 2010052313 A1 | 14-05-2010 | AU 2009312700 A1 | 14-05-2010 |
| | | CA 2742698 A1 | 14-05-2010 |
| | | EP 2361261 A1 | 31-08-2011 |
| | | ES 2479540 T1 | 20-08-2014 |
| | | JP 5745419 B2 | 08-07-2015 |
| | | JP 2012507997 A | 05-04-2012 |
| | | KR 20110123721 A | 15-11-2011 |
| | | US 2012064031 A1 | 15-03-2012 |
| | | WO 2010052313 A1 | 14-05-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2859092 A1
- WO 2010135255 A1
- US 20150104428 A1

### Non-patent literature cited in the description

- **D. A. VIGNALI ; L. W. COLLISON ; C. J. WORK-MAN.** How regulatory T cells work. *Nat Rev Immunol,* 2008, vol. 8, 523-532
- **E. GAMBINERI ; T. R. TORGERSON ; H. D. OCHS.** Immune dysregulation, polyendocrinopathy, enteropathy, and X-linked inheritance (IPEX), a syndrome of systemic autoimmunity caused by mutations of FOXP3, a critical regulator of T-cell homeostasis. *Curr Opin Rheumatol,* 2003, vol. 15, 430-435
- **N. PILAT et al.** Treg-mediated prolonged survival of skin allografts without immunosuppression. *Proc Natl Acad Sci U S A,* 2019, vol. 116, 13508-13516
- **R. D. DANBY et al.** High proportions of regulatory T cells in PBSC grafts predict improved survival after allogeneic haematopoietic SCT. *Bone Marrow Transplant,* 2016, vol. 51, 110-118
- **M. SHARMA et al.** Regulatory T Cells License Macrophage Pro-Resolving Functions During Atherosclerosis Regression. *Circ Res,* 2020, vol. 127, 335-353
- **N. MAREK-TRZONKOWSKA et al.** Therapy of type 1 diabetes with CD4(+)CD25(high) CD127-regulatory T cells prolongs survival of pancreatic islets - Results of one year follow-up. *Clinical Immunology,* 2014, vol. 153, 23-30
- **R. W. M. KEMPKES ; I. JOOSTEN ; H. KOENEN ; X. HE.** Metabolic Pathways Involved in Regulatory T Cell Functionality. *Front Immunol,* 2019, vol. 10, 2839
- **S. LÉTOURNEAU ; C. KRIEG ; G. PANTALEO ; O. BOYMAN.** IL-2- and CD25-dependent immunoregulatory mechanisms in the homeostasis of T-cell subsets. *J Allergy Clin Immunol,* 2009, vol. 123, 758-762
- **T. CHINEN et al.** An essential role for the IL-2 receptor in T(reg) cell function. *Nat Immunol,* 2016, vol. 17, 1322-1333
- **M. VUERICH ; R. P. HARSHE ; S. C. ROBSON ; M. S. LONGHI.** Dysregulation of Adenosinergic Signaling in Systemic and Organ-Specific Autoimmunity. *Int J Mol Sci,* 2019, vol. 20
- **E. CASTILLO-LEON ; S. DELLEPIANE ; P. FIORINA.** ATP and T-cell-mediated rejection. *Curr Opin Organ Transplant,* 2018, vol. 23, 34-43
- **F. MORANDI ; A. L. HORENSTEIN ; R. RIZZO ; F. MALAVASI.** The Role of Extracellular Adenosine Generation in the Development of Autoimmune Diseases. *Mediators Inflamm,* 2018, vol. 7019398
- **M. DURAND et al.** Increased degradation of ATP is driven by memory regulatory T cells in kidney transplantation tolerance. *Kidney Int,* 2018, vol. 93, 1154-1164
- **L. ANTONIOLI ; C. BLANDIZZI ; P. PACHER ; G. HASKÓ.** Immunity, inflammation and cancer: a leading role for adenosine. *Nat Rev Cancer,* 2013, vol. 13, 842-857
- **L. AIRAS ; J. NIEMELÄ ; G. YEGUTKIN ; S. JAL-KANEN.** Mechanism of action of IFN-beta in the treatment of multiple sclerosis: a special reference to CD73 and adenosine. *Ann N Y Acad Sci,* 2007, vol. 1110, 641-648
- **J. NIEMELÄ et al.** IFN-beta regulates CD73 and adenosine expression at the blood-brain barrier. *Eur J Immunol,* 2008, vol. 38, 2718-2726
- **R. S. PERES et al.** Low expression of CD39 on regulatory T cells as a biomarker for resistance to methotrexate therapy in rheumatoid arthritis. *Proc Natl Acad Sci U S A,* 2015, vol. 112, 2509-2514
- **S. BOTTA GORDON-SMITH ; S. URSU ; S. EATON ; H. MONCRIEFFE ; L. R. WEDDERBURN.** Correlation of low CD73 expression on synovial lymphocytes with reduced adenosine generation and higher disease severity in juvenile idiopathic arthritis. *Arthritis Rheumatol,* 2015, vol. 67, 545-554
- **D. J. LEE ; A. W. TAYLOR.** Recovery from experimental autoimmune uveitis promotes induction of antiuveitic inducible Tregs. *J Leukoc Biol,* 2015, vol. 97, 1101-1109
- **L. ANTONIOLI ; C. BLANDIZZI ; B. CSÓKA ; P. PACHER ; G. HASKÓ.** Adenosine signalling in diabetes mellitus--pathophysiology and therapeutic considerations. *Nat Rev Endocrinol,* 2015, vol. 11, 228-241

- **C. BLUME et al.** Autoimmunity in CD73/Ecto-5'-nucleotidase deficient mice induces renal injury. *PLoS One,* 2012, vol. 7, e37100
- **H. TSUKAMOTO et al.** Deficiency of CD73/ecto-5'-nucleotidase in mice enhances acute graft-versus-host disease. *Blood,* 2012, vol. 119, 4554-4564
- **G. D. WHITEHILL et al.** Adenosine Selectively Depletes Alloreactive T Cells to Prevent GVHD While Conserving Immunity to Viruses and Leukemia. *Mol Ther,* 2016, vol. 24, 1655-1664
- **P. TRZONKOWSKI et al.** First-in-man clinical results of the treatment of patients with graft versus host disease with human ex vivo expanded CD4+CD25+CD127- T regulatory cells. *Clin Immunol,* 2009, vol. 133, 22-26
- **N. MAREK-TRZONKOWSKA et al.** inistration of CD4(+)CD25(high)CD127(-) Regulatory T Cells Preserves beta-Cell Function in Type 1 Diabetes in Children. *Diabetes Care,* 2012, vol. 35, 1817-1820
- **P. TRZONKOWSKI et al.** Hurdles in therapy with regulatory T cells. *Sci Transl Med,* 2015, vol. 7, 304ps318
- **M. DI IANNI et al.** Tregs prevent GVHD and promote immune reconstitution in HLA-haploidentical transplantation. *Blood,* 2011, vol. 117, 3921-3928
- **P. HOFFMANN et al.** FOXP3 expression in natural human CD4+CD25+ regulatory T cells upon repetitive in vitro stimulation. *Eur J Immunol,* 2009, vol. 39, 1088-1097
- **A. VISPERAS ; D. A. VIGNALI.** Are Regulatory T Cells Defective in Type 1 Diabetes and Can We Fix Them. *J Immunol,* 2016, vol. 197, 3762-3770
- **J. A. BLUESTONE et al.** Type 1 diabetes immunotherapy using polyclonal regulatory T cells. *Sci Transl Med,* 2015, vol. 7, 315ra189
- **N. MAREK-TRZONKOWSKA et al.** Mild hypothermia provides Treg stability. *Sci Rep,* 2017, vol. 7, 11915
- **N. SAFINIA et al.** Cell Therapy in Organ Transplantation: Our Experience on the Clinical Translation of Regulatory T Cells. *Front Immunol,* 2018, vol. 9, 354
- **A. L. PUTNAM et al.** Expansion of human regulatory T-cells from patients with type 1 diabetes. *Diabetes,* 2009, vol. 58, 652-662
- **K. L. HIPPEN et al.** Massive ex vivo expansion of human natural regulatory T cells (T(regs)) with minimal loss of in vivo functional activity. *Sci Transl Med,* 2011, vol. 3, 83ra41
- **S. LANDMAN et al.** DNA Methyltransferase Inhibition Promotes Th1 Polarization in Human CD4(+)CD25(high) FOXP3(+) Regulatory T Cells but Does Not Affect Their Suppressive Capacity. *J Immunol Res,* 2018, vol. 4973964

- **M. RYBA et al.** Anti-TNF rescue CD4(+)Foxp3(+) regulatory T cells in patients with type 1 diabetes from effects mediated by TNF. *Cytokine,* 2011, vol. 55, 353-361
- **Z. M. LIU ; K. P. WANG ; J. MA ; S. GUO ZHENG.** The role of all-trans retinoic acid in the biology of Foxp3+ regulatory T cells. *Cell Mol Immunol,* 2015, vol. 12, 553-557
- **M. PIKULA ; N. MAREK-TRZONKOWSKA ; A. WARDOWSKA ; A. RENKIELSKA ; P. TRZONKOWSKI.** Adipose tissue-derived stem cells in clinical applications. *Expert Opin Biol Ther,* 2013, vol. 13, 1357-1370
- **V. SÉNÉCAL et al.** Production of IL-27 in multiple sclerosis lesions by astrocytes and myeloid cells: Modulation of local immune responses. *Glia,* 2016, vol. 64, 553-569
- **X. T. LIMA et al.** Frequency and characteristics of circulating CD4(+) CD28(null) T cells in patients with psoriasis. *Br J Dermatol,* 2015, vol. 173, 998-1005
- **Q. MU ; H. ZHANG ; X. M. LUO.** SLE: Another Autoimmune Disorder Influenced by Microbes and Diet. *Front Immunol,* 2015, vol. 6, 608
- **W. ORENT et al.** Rheumatoid arthritis-associated RBPJ polymorphism alters memory CD4+ T cells. *Hum Mol Genet,* 2016, vol. 25, 404-417
- **S. GUPTA.** Immunotherapies in diabetes mellitus type 1. *Med Clin North Am,* 2012, vol. 96, 621-634
- **I. RAMA ; J. M. GRINYÓ.** Malignancy after renal transplantation: the role of immunosuppression. *Nat Rev Nephrol,* 2010, vol. 6, 511-519
- **Á. PRÓKAI et al.** Calcineurin-inhibition Results in Upregulation of Local Renin and Subsequent Vascular Endothelial Growth Factor Production in Renal Collecting Ducts. *Transplantation,* 2016, vol. 100, 325-333
- **T. BERNEY ; A. SECCHI.** Rapamycin in islet transplantation: friend or foe. *Transpl Int,* 2009, vol. 22, 153-161
- **K. ZHAO et al.** Dynamic regulation of effector IFN-γ-producing and IL-17-producing T cell subsets in the development of acute graft-versus-host disease. *Mol Med Rep,* 2016, vol. 13, 1395-1403
- **R. A. PANETTIERI, JR. ; R. COVAR ; E. GRANT ; E. V. HILLYER ; L. BACHARIER.** Natural history of asthma: persistence versus progression-does the beginning predict the end. *J Allergy Clin Immunol,* 2008, vol. 121, 607-613
- **Q. TANG ; J. A. BLUESTONE.** Regulatory T-cell therapy in transplantation: moving to the clinic. *Cold Spring Harb Perspect Med,* 2013, vol. 3